# EUROPEAN PATENT APPLICATION

(11) **EP 1 180 352 A1**
(43) Date of publication of application: **20.02.2002**
(21) Application number: 00911335.8
(22) Date of filing: 24.03.2000
(51) Int. Cl.: A61G 7/015, A47C 17/04

(54) **DEVICE FOR MOVING BODY**

(30) Priority: 25.03.1999 JP 8157799; 29.03.1999 JP 8659199; 12.04.1999 JP 10405399; 29.07.1999 JP 21470899; 29.07.1999 JP 21470999
(71) Applicant: MATSUSHITA SEIKO CO.LTD., Osaka 536-8555 (JP); Soejima, Noboru, Tokyo 108-0073 (JP)
(72) Inventor: SOEJIMA, Noboru, Tokyo 108-0073 (JP); KATOU, Kenshi, Nagoya-shi, Aichi 463-0002 (JP); NAKAMURA, Masataka, Ichinomiya-shi, Aichi 491-0831 (JP); MIYAMOTO, Hiroyuki, Sanda-shi, Hyogo 669-1544 (JP); MIYAHARA, Masayoshi, Kasugai-shi, Aichi 486-0811 (JP); KURIHARA, Michio, Kasugai-shi, Aichi 487-0032 (JP); GOTOU, Hiroichi, Kasugai-shi, Aici 487-0006 (JP); SHINDOU, Hirofumi, Kasugai-shi, Aichi 487-0032 (JP); TANAGO, Hiroshi, Kasugai-shi, Aichi 480-0304 (JP); ISHIHARA, Yuji, Hirakata-shi, Osaka 573-1107 (JP); MATSUMOTO, Kenji, Kasugai-shi, Aichi 486-0826 (JP)
(74) Representative: Körfer, Thomas, Dipl.-Phys.
(86) International application number: JP0001806
(87) International publication number: WO0057828

(57) **Abstract**

The present invention relates to an apparatus for moving a body, wherein a phantom axis passing a head to a center of legs of the body is defined as a vertical axis, a phantom axis passing an abdomen in the longitudinal direction is defined as a longitudinal axis, and a phantom axis passing the abdomen in the lateral direction is defined as a lateral axis, and motions around these three axes are carried out individually or in combination. According to the invention, it is possible to support, twist and vertically move a body suitably for various individual somatotype and posture.

## Description

### TECHNICAL FIELD

The present invention relates to an apparatus and a method for moving a human body for bringing a bad physical condition into an excellent physical condition, or for keeping the excellent physical condition so that user can spend healthy and comfortable life. The invention also relates to a physical condition adjusting method and a physical condition adjusting apparatus. The apparatus for moving a body or the adjusting apparatus for adjusting the physical condition includes a bed used in a bedroom or hospital and capable of moving physically in accordance with a human body, or capable of changing attitude. The present invention also relates to a driving method of such a bed, and a bed operating apparatus for operating such a bed.

### BACKGROUND TECHNIQUE

To adjust a physical condition of human body or keep a healthy condition, various healthy methods have been tried conventionally.

In generally, in healthy methods, mental and body can not be separated, but health care can be divided into mental care and physical care.

The mental methods are approaches to sensibilities such as fun and pleasure, but here, health care for body is conceived except medication or diet.

That is, the health care for body includes walking, radio exercise, jogging, stretching and the like, and health care using a third party's power includes aligning massage, bathing and the like.

In the aligning massage using the third party's power, portions of a human body are pressed, expanded, contracted, warmed or cooled by man power or machine, thereby promoting the circulation of blood, promoting metabolism, and removing fatigue of muscles.

The aligning massage corrects distortion of a body to form a well-balanced body, thereby enhancing healthy condition of keeping the healthy condition.

There is a conventional bed described in Japanese Patent Applications Laid-open Nos.H4-307004 and H8-47437.

The bed described in Japanese Patent Application Laid-open No.H4-307004 supplies twisting swinging motion to a human body or organ so as to move muscle or organ. The bed described in Japanese Patent Application Laid-open No.H8-47437 supplies vertical swinging motion of 1/f of a bottom of the bed.

A clinical detection system described in Japanese Patent Application Laid-open No.H10-55491 includes a load sensor interposed between a column and a floor of a bed to collectively manage whether a man exists on the bed.

Generally, in the aligning massage, portions of a human body are collectively massaged, pushed, expanded or contracted, and human power or energy is required for such movements. The same can be said when bend of human body is corrected to form well-balanced body.

A person who is massaged feels comfortable, but may feel pain due to excessive power in some cases. Therefore, whenmassage is carried out, it is important to massage such that a degree of power is suitable for the particular person on a case-by-case basis. Especially when an old person is massaged, care is required.

In some conventional beds, an entire surface of amat support stage on which a man lies swings, it is difficult to adjust the swinging motion around a loin to individual persons having different bodily shape and attitude, and it is required that an upper surface of the bed can freely be adjusted to individual attitudes.

There are different sleeping attitudes depending upon individuals, and simple inclination or twisting can not cover all the attitudes. Therefore, it is required to change various portions of the upper surface of the bed into different states.

Further, it is not easy to take a deep sleep only with comfortable bed room or bed due to deterioration in living environment, and it is required not to guide him or her into sleep, but also to set comfort during sleep and at the time of rising.

Especially in recent years, desk work in the office using personal computer is popular, and people keeps the same attitude for a long time and thus, stress builds up easily. Further, many people are suffering from insomnia.

Further, it is danger for people having encephalopathy or low blood pressure to rise abruptly, and there is a problem that it takes a long time even for a normal person to wake up completely after rising.

There are many people who always have pain or stress in anywhere in his or her body due to habitual motion or attitude, and in recent years, many people are concerned about effect of stress exerted on body.

Every person thinks that if user can move body more, user can do anything not only sports or dances.

In an operating apparatus used for such a bed, in a state in which the operating apparatus is put on the bed, an operation input switch is pushed by a body or comforter during sleep, and there is a problem that the switch is operated irrespective of a will of the user. The conventional operating apparatus having a malfunction preventing switch separately from the operation switch, there is a problem that the operation is complicated, and the operability is inferior.

In many of the operating apparatus having an illumination function, since display means or operating switch is highly illuminate when it is used or not, when it is used as a bed operating apparatus, even when a room illumination is darkened at the time of sleeping, if the illumination of the operating switch is lit, this prevent the sleeping.

When the bed is operated, since a load applied to driving means is varied due to different in weight of the user or difference in sleeping attitude, it is difficult to control the driving speed, and it is difficult to always keep moving speed which was set during the design phase. Especially when there is abrupt motion or delay with respect to the speed change, it is impossible to give satisfaction to a user.

It is an object of the present invention to provide an apparatus and method for moving a human body which does not exert a great deal of energy or undue force, which is safe for old person, and which has effect as health method, and to provide a body adjusting method and adjusting apparatus.

Further, it is another object of the invention to provide a bed capable of supporting a human body in a suitable manner for bodily shape and attitude of individual when the user relaxed on the bed, capable of swinging or vertically moving in normal and reverse directions a bottom which was divided into a plurality of pieces, and capable of obtaining comfortable sleep.

Further, it is another object of the invention to provide a driving method of a bed capable of moving three axes of a body to relax vertebra errector, and capable of overcoming stress by relaxing movement which gives precedence to parasympathetic nervous system.

It is another object of the invention to provide a driving method of a bed which gives precedence to parasympathetic nervous system and induces sleeping.

It is another object of the invention to provide a driving method of a bed which allows a user to wake up cleanly within a short time by waking up movement, and which allows, for example, people having encephalopathy or low blood pressure to wake up cleanly within a short time safely.

It is another object of the invention to provide a driving method which allows a user to find fine movement of body to exploit ability that the user originally has, which reduces the stress of thorax, and improves breathing.

It is another object of the invention to provide a driving methodwhich promotes the circulation of blood of loin by repeating stretch of psoas, vertebra erector, and the like, and stress is eliminated, thereby eliminating fatigue of loin, and preventing backache.

It is another object of the invention to provide a driving method which moves at suitable speed or angle for a user so that effect can sufficiently be exhibited for people having different ages and flexibility.

It is another object of the invention to provide a bed operating apparatus which prevents an operating input switch from being pushed by a body or comforter even if a user slept in a state in which the operating apparatus is placed on the bed, and which prevents the bed from being operating against a will of the user.

It is another object of the invention to provide a bed operating apparatus in which only when a user takes the operating apparatus in his or her hand, the apparatus is illuminated with high illumination so that sleeping is not hindered and the operability is enhanced.

It is another object of the invention to provide a bed operating apparatus in which illumination is changed, the illumination is gradually changed so that a user is less prone to feel glare, and the using comfort can be enhanced.

It is another object of the invention to provide a bed in which various operations can be carried out smoothly even if a load condition such as weight of a user is varied.

It is another object of the invention to provide a bed in which can move at set acceleration without affecting the using condition and constant satisfaction and feeling of security can always be given to a user.

It is another object of the invention to provide a bed capable of avoiding breakdown caused by excessive load condition or breakdown caused by sandwiching of comforter or the like.

### DISCLOSURE OF THE INVENTION

In an apparatus for moving a body of the invention described in claim 1, a phantom axis passing a head to a center of legs of the body is defined as a vertical axis, a phantom axis passing an abdomen in the longitudinal direction is defined as a longitudinal axis, and a phantom axis passing the abdomen in the lateral direction is defined as a lateral axis, and motions around these three axes are carried out individually or in combination.

In an apparatus for moving a body of the invention described in claim 2, a phantom axis passing a head to a center of legs of the body is defined as a vertical axis, and a phantom axis passing an abdomen in the longitudinal direction is defined as a longitudinal axis, and motions around these two axes are carried out individually or in combination.

In an apparatus for moving a body of the invention described in claim 3, a phantom axis passing a head to a center of legs of the body is defined as a vertical axis, arid a phantom axis passing the abdomen in the lateral direction is defined as a lateral axis, and motions around these two axes are carried out individually or in combination.

In an apparatus for moving a body of the invention described in claim 4, a phantom axis passing an abdomen in the longitudinal direction is defined as a longitudinal axis, and a phantom axis passing the abdomen in the lateral direction is defined as a lateral axis, and motions around these two axes are carried out individually or in combination.

According to the invention described in claims 1 to 4, it is possible to support, twist and vertically move a body suitably for various individual somatotype and posture.

In an apparatus for moving a body of the invention described in claim 5, a phantom axis passing a top of a head to soles of legs of the body standing upright or lying face up or down is defined as a vertical axis, a phantom axis passing an abdomen in the longitudinal direction is defined as a longitudinal axis, and a phantom axis passing the abdomen in the lateral direction is defined as a lateral axis, and a motions around these three phantom axes are regularly carried out.

According to the invention, in a state the body is standing upright or lying face up or down, the motions around the three phantom axes are carried out regularly, and various motion can be carried out for the entire body.

In an apparatus for moving a body of the invention described in claim 6, a phantom axis passing a top center of a head to a center of soles of legs of the body standing upright or lying face up or down is defined as a vertical axis, a phantom axis passing a bendable abdomen in the longitudinal direction is defined as a longitudinal axis, and a phantom axis passing the bendable abdomen in the lateral direction is defined as a lateral axis, a turning motion around said vertical axis, and a laterally swinging motion around the longitudinal axis and a lying up and down motion around the lateral axis are combined.

According to this invention, in a state the body is standing upright or lying face up or down, a turning motion around said vertical axis, a laterally swinging motion around the longitudinal axis and a lying up and down motion around the lateral axis can be combined.

In an apparatus for moving a body of the invention described in claim 7, a phantom axis passing atop center of a head to a center of sitting posture of the sitting body is defined as a vertical axis, a phantom axis passing an abdomen in the longitudinal direction is defined as a longitudinal axis, and a phantom axis passing the abdomen in the lateral direction is defined as a lateral axis, and motions around these three phantom axes are regularly carried out.

According to this invention, motions around these three phantom axes are regularly carried out in a state in which the body is sitting.

In an apparatus for moving a body of the invention described in claim 8, a phantom axis passing a top center of a head to a center of sitting posture of the sitting body is defined as a vertical axis, a phantom axis passing a bendable abdomen in the longitudinal direction is defined as a longitudinal axis, and a phantom axis passing the bendable abdomen in the lateral direction is defined as a lateral axis, and a turning motion around the vertical axis, a laterally swinging motion around the longitudinal axis, and a lying up and down motion around the longitudinal axis are combined.

According to this invention, a turning motion around the vertical axis, a laterally swinging motion around the longitudinal axis, and a lying up and down motion around the lateral axis are combined in a state in which the body is sitting.

In an apparatus for moving a body of the invention described in claim 9, a phantom axis passing through a head to a center of legs of the body is defined as a vertical axis, a phantom axis passing through an abdomen surface to a back surface in a longitudinal direction is defined as a longitudinal axis, and a phantom axis passing through a body side surface in a lateral direction is defined as a lateral axis, a rotating motion around the vertical axis based on at least one origin from a lumbar vertebrae, a chest thoracic vertebrae and a cervical vertebra, a laterally swinging motion around a phantom axis in which a position of the lateral axis is at least one point from a loin to a chest and cervix, and a lying up and down motion around a phantom axis in which a position of the longitudinal axis is at least one point from the loin to the chest and the cervix, are carried out individually or in combination.

According to this invention, a rotating motion around the vertical axis based on at least one origin from a lumbar vertebrae, a chest thoracic vertebrae and a cervical vertebra, a laterally swinging motion around a phantom axis in which a position of the longitudinal axis is at least one point from a loin to a chest and cervix, and a lying up and down motion around a phantom axis in which a position of the longitudinal axis is at least one point from the loin to the chest and the cervix, are carried out individually or in combination.

In an apparatus for moving a body of the invention described in claim 10, in the apparatus of claim 9, when at least two of the turning motion, the laterally swinging motion and the lying up and down motion are carried out continuously, a position of an origin of the vertical axis, a position of the longitudinal axis and a position of the lateral axis are fixed or moved.

According to this invention, when at least two of the turning motion, the laterally swinging motion and the lying up and down motion are carried out continuously, a position of an origin of the vertical axis, a position of the longitudinal axis and a position of the lateral axis are fixed or moved. Since the phantom axis position can be changed, it is possible to move the body differently.

A bed of the invention described in claim 11 for moving a body uses an apparatus for moving a body described in any one of claims 1 to 10.

According to this invention, the apparatus described in claims 1 to 10 is applied to the bed, and sleeping effect and waking up effect are provided.

In a body adjusting method of the invention described in claim 12, a phantom axis passing a head to a center of legs of the body is defined as a vertical axis, a phantom axis passing an abdomen in the longitudinal direction is defined as a longitudinal axis, and a phantom axis passing the abdomen in the lateral direction is defined as a lateral axis, and motions around these three axes are carried out individually or in combination, and the method adjusts the body so that distortion or unbalance of the body is eliminated, circulation of blood is promoted, and the body is relaxed.

According to this invention, motions around these three axes are carried out individually or in combination, and the method adjust the body so that distortion or unbalance of the body is eliminated, circulation of blood is promoted, and the body is relaxed.

In a body adjusting method of the invention described in claim 13, a phantom axis passing a top of a head to soles of legs of the body standing upright or lying face up or down is defined as a vertical axis, a phantom axis passing an abdomen in the longitudinal direction is defined as a longitudinal axis, and a phantom axis passing the abdomen in the lateral direction is defined as a lateral axis, and a motions around these three phantom axes are regularly carried out so that distortion or unbalance of the body is eliminated, circulation of blood is promoted, and the body is relaxed.

According to this invention, in a state in which the body is standing up or lying up, a motions around these three phantom axes are regularly carried out so that distortion or unbalance of the body is eliminated, circulation of blood is promoted, and the body is relaxed.

In a body adjusting method of the invention described in claim 14, a phantom axis passing a top center of a head to a center of soles of legs of the body standing upright or lying face up or down is defined as a vertical axis, a phantom axis passing a bendable abdomen in the longitudinal direction is defined as a longitudinal axis, and a phantom axis passing the bendable abdomen in the lateral direction is defined as a lateral axis, and a turning motion around said vertical axis, a laterally swinging motion around the longitudinal axis and a lying up and down motion around the lateral axis are combined so that distortion or unbalance of the body is eliminated, circulation of blood is promoted, and the body is relaxed.

According to this invention, in a state in which the body is standing up or lying up, a turning motion around said vertical axis, a laterally swinging motion around the longitudinal axis and a lying up and down motion around the lateral axis are combined so that distortion or unbalance of the body is eliminated, circulation of blood is promoted, and the body is relaxed.

In a body adjusting method of the invention described in claim 15, a phantom axis passing a top center of a head to a center of sitting posture of the sitting body is defined as a vertical axis, a phantom axis passing an abdomen in the longitudinal direction is defined as a longitudinal axis, and a phantom axis passing the abdomen in the lateral direction is defined as a lateral axis, and motions around these three phantom axes are regularly carried out so that distortion or unbalance of the body is eliminated, circulation of blood is promoted, and the body is relaxed.

According to this invention, in the sitting state, motions around these three phantom axes are regularly carried out so that distortion or unbalance of the body is eliminated, circulation of blood is promoted, and the body is relaxed.

In a body adjusting method of the invention described in claim 16, a phantom axis passing a top center of a head to a center of sitting posture of the sitting body is defined as a vertical axis, a phantom axis passing a bendable abdomen in the longitudinal direction is defined as a longitudinal axis, and a phantom axis passing the bendable abdomen in the lateral direction is defined as a lateral axis, and a turning motion around the vertical axis, a laterally swinging motion around the longitudinal axis, and a lying up and down motion around the lateral axis are combined so that distortion or unbalance of the body is eliminated, circulation of blood is promoted, and the body is relaxed.

According to this invention, distortion or unbalance of the body is eliminated, circulation of blood is promoted, and the body is relaxed.

In a body adjusting method of the invention described in claim 17, a phantom axis passing through a head to a center of legs of the body is defined as a vertical axis, a phantom axis passing through an abdomen surface to a back surface in a longitudinal direction is defined as a longitudinal axis, and a phantom axis passing through a body side surface in a lateral direction is defined as a lateral axis, a rotating motion around the vertical axis based on at least one origin from a lumbar vertebrae, a chest thoracic vertebrae and a cervical vertebra, a laterally swinging motion around a phantom axis in which a position of the longitudinal axis is at least one point from a loin to a chest and cervix, and a lying up and down motion around a phantom axis in which a position of the longitudinal axis is at least one point from the loin to the chest and the cervix, are carried out individually or in combination so that distortion or unbalance of the body is eliminated, circulation of blood is promoted, and the body is relaxed.

According to this invention, distortion or unbalance of the body is eliminated, circulation of blood is promoted, and the body is relaxed.

In a body adjusting method of the invention described in claim 18, in the method of claim 17, a turning motion around the vertical axis having an origin which is at least one of the loin, the chest and cervix, a laterally swinging motion around a phantom axis having a position of the longitudinal axis which is at least one of the loin, the chest and cervix, and a lying up and down motion around a phantom axis having a position of the longitudinal axis which is at least one of the loin, the chest and cervix, are carried out individually or in combination, and when two or more of the body motions are carried out continuously, a position of the origin of the vertical axis, a position of the longitudinal axis and a position of the lateral axis are fixed or moved every time, so that distortion or unbalance of the body is eliminated, circulation of blood is promoted, and the body is relaxed.

According to this invention, distortion or unbalance of the body is eliminated, circulation of blood is promoted, and the body is relaxed.

An adjusting apparatus of the invention described in claim 19 carries out a body adjusting method described in any one of claims 12 to 18.

According to this invention, these body adjusting methods can be carried out.

In a bed of the invention described in claim 20 having a bottom which is divided into a plurality of members and in which the divided members can move such as to form different slants, the bed includes swinging mechanism means for giving a twisting motion to at least one of the members of the bottom.

According to this invention, a portion of the body can be twisted, and various twisting effects can be given to the body.

In a bed of the invention described in claim 21, in the bed of claim 20, the member of the bottom to which the twisting motion is given by the swinging mechanism means is a back rising bottom for supporting a back.

According to this invention, especially upper half body can variously be twisted.

In a bed of the invention described in claim 22, in the bed of claim 20 or 21, an inclination angle of a back rising bottom for supporting a back can be changed.

According to this invention, twist and stretch can be combined for the body.

In a bed of the invention described in claim 23, in the bed of claim 20, the member of the bottom to which the twisting motion is given by the swinging mechanism means is a leg rising bottom for supporting a leg.

According to this invention, especially lower half body can variously be twisted.

In a bed of the invention described in claim 24, in the bed of claim 20 or 23, an inclination angle of a leg rising bottom for supporting a leg can be changed.

According to this invention, twist and stretch can be combined for the body.

In a bed of the invention described in claim 25 having a bottom which is divided into a plurality of members and in which the divided members can move such as to form different slants, at least one of the members of the bottom constitutes a back rising bottom, at least one of the members of the bottom constitutes a leg rising bottom, and inclination angles of the back rising bottom and leg rising bottom can be changed.

According to this invention, the upper and lower half bodies can be stretched.

In a bed of the invention described in claim 26 in the bed of claim 25, at least two of the members of the bottom constitute the leg rising bottom, and at least two of the members constituting the leg rising bottom form a mountain-like slant such as to bring a knee of a body.

According to this invention, by bringing up the knee, it is easy to stand up from the bed, and load on loin can be reduced.

In a bed of the invention described in claim 27, in the bed of claim 25, further comprises back rising driving means for changing the inclination angle of the back rising bottom, and leg rising driving means for changing the inclination angle of the leg rising bottom.

According to this invention, inclination angles of the back rising bottom and the leg rising bottom can separately be changed, and stretching effect for the upper and lower half bodies can be enhanced.

In a bed of the invention described in claim 28, in the bed of claim 27, the back rising driving means changes an inclination angle of the back rising bottom while twisting the back rising bottom.

According to this invention, the upper half body of the body can be twisted and stretched in combination.

In a bed of the invention described in claim 29, in the bed of claim 27, the legrising driving means changes an inclination angle of the leg rising bottom while twisting the leg rising bottom.

According to this invention, the lower half body of the body can be twisted and stretched in combination.

A bed of the invention described in claim 30, in the bed of claim 27, further comprises display means for displaying a twist angle or inclination angle of the back rising bottom or the leg rising bottom.

According to this invention, the bed is operated while confirming the motion state, this eliminates jitter of the user, and the bed can be operated safely.

A bed of the invention described in claim 31, in the bed of claim 27, further comprises an operating portion for controlling a driving operation of the back rising driving means and the leg rising driving means.

According to this invention, the user can operate the operating means in accordance with the user's will.

A bed of the invention described in claim 32, in the bed of claim 27, further comprises storing means for storing a twist angle or an inclination angle of the back rising bottom or the leg rising bottom.

According to this invention, the user can repeatedly utilize motion in accordance with the user's will.

A bed of the invention described in claim 33 comprises a bottom which is divided into a plurality of members which can move such as to form different slants, a body motion detector for detecting a motion of a body existing on the bottom, sleeping state judging means for judging a sleeping state by a signal from the body motion detector, driving means for moving at least one member of the bottom, and driving control means for controlling a driving of the driving means, the driving control means controls the driving of the driving means by a signal from the sleeping state judging means.

According to this invention, it is possible to select and operate comfortable motion depending upon the sleeping state.

In a bed of the invention described in claim 34, in the bed of claim 33, the driving control means moves the driving means after a predetermined time is elapsed from an instant when the driving control means received a sleeping state completion judging signal from the sleeping state judging means.

According to this invention, the waking up effect can be enhanced.

In a bed of the invention described in claim 35, in the bed of claim 33, if the driving control means received a sleeping state judging signal from the sleeping state judging means, the driving control means allows the driving means to move such as to return the back rising bottom and the leg rising bottom to a horizontal state or an inclination state which was previously set as a slant in the sleeping state

According to this invention, tossing and turning are allowed during sleeping, feel of constraint is eliminated.

A bed of the invention described in claim 36 comprises a bottom which is divided into a plurality of members which can move such as to form different slants, a twisting driving means for twisting at least one of the members of the bottom, a plurality of load sensors for detecting weights of portions of a body existing on the bottom, a plurality of output means for outputting a detection signal detected by the load sensor, and comparing means for comparing output signal value from the output means, the twisting driving means twists at least one of the members of the bottom based on a comparison signal from the comparing means.

According to this invention, it is possible to adjust the twist of the bed while taking unbalance of the body into account.

A bed of the invention described in claim 37 comprises a bottom which is divided into a plurality of members which can move such as to form different slants, a driving means for moving at least one of the members of the bottom, a load sensor for detecting a weight of a body existing on the bottom, output means for outputting a detection signal detected by the load sensor, and a time setting means for setting a predetermined time, when a signal is not output from the output means within time set by the time setting means, this state is judged as a sleeping state, and an output signal from the driving means is changed.

According to this invention, by changing the motion content before and after the sleep, the deep sleep or start-sleeping effect can be enhanced.

In a bed of the invention described in claim 38, in the bed of claim 37, when a signal is not output from the output means within time set by the time setting means, a motion state is released, or the state is returned to a state which was previously set as a sleeping state.

According to this invention, it is possible to eliminate the feeling of constraint in the sleeping state.

A bed of the invention described in claim 39 comprises a bottom which is divided into a plurality of members which can move such as to form different slants, a twisting driving means for moving at least one of the members of the bottom, and timer means for setting a predetermined time, after time set by the timer means was elapsed, an output signal from the driving means is changed.

According to this invention, the user can change the motion content to meet the user's will.

In a bed of the invention described in claim 40, in the bed of claim 39, after time set by the timer means was elapsed, a motion state is released, or the state is returned to a state which was previously set as a sleeping state.

According to this invention, it is possible to eliminate the feeling of constraint in the sleeping state.

A bed of the invention described in claim 41 comprises a bottom which is divided into a plurality of members which can move such as to form different slants, a driving means for moving at least one of the members of the bottom, a load sensor for detecting a weight of a body existing on the bottom, output means for outputting a detection signal detected by the load sensor, and weight judging means for judging whether there is a body based on a signal from the output means, when the existence of the body can not be confirmed by the weight judging means, the driving means is not moved.

According to this invention, if existence of a body can not be confirmed by the weight judging means, the driving means can not be operated, and this prevents erroneous operation.

A bed of the invention described in claim 42, in the claim 41, further comprises weight setting means for storing weight of a bed user, the weight judging means judges the bed user from a weight detected by the load sensor and a weight set by the weight setting means, and when the weight judging means can not confirm the bed user, the driving means is not moved.

According to this invention, the erroneous operation can be prevented more effectively.

A bed of the invention described in claim 43 comprises a bottom divided into a plurality of members, twisting mechanism means for twisting a back rising bottom supporting a back of the bottom, a plurality of load sensor disposed between legs of the bottom and a floor surface, weight judging means for calculating a weight of each portion based on a detection signal detected by the load sensors, weight comparing means for comparing weights of portions calculated by the weight judging means, deviation amount converting means for converting a weight difference obtained by comparison of the weight comparing means into a deviation amount with respect to a center of the bed, twisting motion correcting means for correcting a twisting amount based on the deviation amount, and driving means for driving the twisting mechanism means based on twist motion corrected by the twisting motion correcting means.

According to this invention, twist motion can be given in accordance with distortion of the body of the user.

In a bed driving method of the invention described in claim 44 having a back rising bottom for supporting a back, the back rising bottom is vertically moved in a predetermined angle range with respect to a horizontal plane while twisting the back rising bottom.

According to this invention, the twist motion and stretching motion are carried and the relaxing effect can be enhanced.

In a bed driving method of the invention described in claim 45, in the method of claim 44, a twist motion and a vertical motion given to the back rising bottom are carried out in predetermined rhythm.

According to this invention, the relaxing effect can be enhanced.

In a bed driving method of the invention described in claim 46, in the method of claim 44, a twist motion given to the back rising bottom is carried out twice or less/second.

According to this invention, it is possible to carry out comfortable motion without giving uncomfortable feeling to the user.

In a bed driving method of the invention described in claim 47, in the method of claim 44, a twist motion given to the back rising bottom is carried out at an angle of 15° or more at maximum with respect to a horizontal plane, and a vertical motion is carried out at an angle of 5° at maximum with respect to the horizontal plane.

According to this invention, the relaxing effect can be enhanced.

In a bed driving method of the invention described in claim 48, in the method of claim 44, a twist motion and a vertical motion given to the back rising bottom are repeatedly carried · out while setting a maximum variation of one time is equal to or less than 10°.

According to this invention, the user can sleep easier by fine motion.

In a bed driving method of the invention described in claim 49, a twist motion given to the back rising bottom is carried out at an angle of 20° or less with respect to a horizontal plane, and a vertical motion is carried out at an angle of 20° or less with respect to the horizontal plane.

According to this invention, the user can sleep easier by fine motion.

In a bed driving method of the invention described in claim 50, a twist motion given to the back rising bottom is carried out at a speed of twice or less/second for a predetermined time and then, the twist motion is carried out at a speed higher than twice/second.

According to this invention, waking up effect can be enhanced.

In a bed driving method of the invention described in claim 51, in the method of claim 44, a twist emotion given to the back rising bottom is carried out while gradually increasing speed from twice or less/second to twice or more/second.

According to this invention, waking up effect can be enhanced.

In a bed driving method of the invention described in claim 52, in the method of claim 44, an angle of a twist motion with respect to a horizontal plane and an angle of a vertical motion with respect to a horizontal plane are gradually increased.

According to this invention, waking up effect can be enhanced.

In a bed driving method of the invention described in claim 53, in the method of claim 44, a twist motion and a vertical motion given to the back rising bottom are carried out at different timings.

According to this invention, finding effect can be enhanced.

In a bed driving method of the invention described in claim 54 having a back rising bottom for supporting a back, the back rising bottom is brought upward through a predetermined angle and then, the back rising bottom is lowered while twisting the back rising bottom.

According to this invention, loin fatigue can be eliminated and lumbago can be prevented.

In a bed driving method of the invention described in claim 55, in the method of claim 54, the predetermined angle for bringing the back rising bottom upward is 20° or more with respect to a horizontal plane.

According to this invention, loin fatigue can be eliminated and lumbago can be prevented.

In a bed driving method of the invention described in claim 56, in the method of claim 54, the back rising bottom is repeatedly brought upward and downward.

According to this invention, loin fatigue can be eliminated and lumbago can be prevented.

In a bed driving method of the invention described in claim 57 having a back rising bottom for supporting a back and a leg rising bottom for supporting a leg, the back rising bottom and the leg rising bottom are brought upward through a predetermined angle and then, the back rising bottom and the leg rising bottom are lowered while twisting the back rising bottom.

According to this invention, loin fatigue can be eliminated and lumbago can be prevented.

In a bed driving method of the invention described in claim 58, in the method of claim 57, a total of the predetermined angle for bringing the back rising bottom and the predetermined angle for bringing the leg rising bottom with respect to a horizontal plane is 20° or more.

According to this invention, loin fatigue can be eliminated and lumbago can be prevented.

In a bed driving method of the invention described in claim 59, in the method of claim 57, the back rising bottom and the leg rising bottom are repeatedly brought upward and downward.

According to this invention, loin fatigue can be eliminated and lumbago can be prevented.

In a bed driving method of the invention described in claim 60, in the method of claims 44 to 59, a speed for bringing the back rising bottom upward or downward can be selected from preset speeds, or can be set in a preset speed range.

According to this invention, exercise effect can be enhanced by each motion in accordance with the user's liking.

In a bed driving method of the invention described in claim 61, in the method of claims 57 to 59, a speed for bringing the leg rising bottom upward or downward can be selected from preset speeds, or can be set in a preset speed range.

According to this invention, exercise effect can be enhanced by each motion in accordance with the user's liking.

In a bed driving method of the invention described in claim 62, in the method of claims 44 to 59, an angle for bringing the back rising bottom upward or downward can be selected from preset angles, or can be set in a preset angle range.

According to this invention, exercise effect can be enhanced by each motion in accordance with the user's liking.

In a bed driving method of the invention described in claim 63, in the method of claims 57 to 59, an angle for bringing the leg rising bottom upward or downward can be selected from preset angles, or can be set in a preset angle range.

According to this invention, exercise effect can be enhanced by each motion in accordance with the user's liking.

In a bed driving method of the invention described in claim 64, having a back rising bottom for supporting a back or a leg rising bottom for supporting a leg, the back rising bottom or the leg rising bottom is rotated through a predetermined angle around a phantom longitudinal axis passing an abdomen of a body lying on a bed in a longitudinal direction.

According to this invention, twisting effect for the body can be enhanced by the rotating motion.

In a bed driving method of the invention described in claim 65, in the method of claim 64, a rotation angle or a rotation direction of the back rising bottom or the leg rising bottom can be selected from preset angles, or can be set in a preset angle range.

According to this invention, exercise effect can be enhanced by each motion in accordance with the user's liking.

In a bed driving method of the invention described in claim 66 having a back rising bottom for supporting a back and a leg rising bottom for supporting a leg, the back rising bottom and the leg rising bottom carry out a twist motion around a phantom vertical axis passing through a head and a center of legs of a body lying on a bed, a turning motion around a phantom longitudinal axis passing through an abdomen of the body lying on the bed in a longitudinal direction, and a vertical motion around a phantom lateral axis passing through the abdomen of the body lying on the bed in a lateral direction, a plurality of motion patterns comprising the twist motion, the turning motion and the vertical motion individually or in combination are previously set, the order of these motion patterns is changed, or a speed, an angle or a direction of each of the motion patterns can be changed.

According to this invention, exercise effect can be enhanced by each motion in accordance with the user's liking.

A bed operating apparatus of the invention described in claim 67 comprises a box-like outline, operation will detecting means provided on at least one surface of the outline for detecting operation will, operation content detecting means provided on at least one surface of the outline for detecting operation content, and operation instruction determining means for outputting the operation content when operation will is detected by the operation will detecting means and operation content is detected by the operation content detecting means.

According to this invention, erroneous operation can effectively be prevented, and the bed can be operated easily.

In a bed operating apparatus according to claim 67, wherein said operation will detecting means is provided on each of two opposed side surfaces of said outline.

According to this invention, erroneous operation can effectively be prevented.

In a bed operating apparatus of the invention described in claim 69, in the apparatus of claim 67, the operation will detecting means and the operation content detecting means are provided on different surfaces of the outline.

According to this invention, erroneous operation can effectively be prevented.

A bed operating apparatus of the invention described in claim 70, in the bed of claim 67, further comprises display means for displaying the operation content or motion state of the bed, and illumination changing means for changing illumination of the display means, the illumination changing means changes illumination to high level as compared before the operation will is detected, when the operating will is detected by the operation will detecting means.

According to this invention, the operability can be enhanced without hindering the sleep.

In a bed operating apparatus of the invention described in claim 71, in the apparatus of claim 70, after a predetermined time was elapsed from an instant when the operating will was detected by the operation will detecting means, or after a predetermined time was elapsed from an instant when the operating will was not detected by the operation will detecting means, or when the operating will was not detected by the operation will detecting means, the illumination is changed to low level.

According to this invention, the operability can be enhanced and the sleep is not hindered.

In a bed operating apparatus of the invention described in claim 72, in the apparatus of claim 70 or 71, the illumination is gradually changed.

According to this invention, even if the operation apparatus is used in a state in which the room illumination is lowered when the user sleeps, the illumination is gradually increased. Therefore, the user is less prone to feel glare, and the user can use the bed more comfortably.

A bed driving apparatus of the invention described in claim 73 comprises storing means which previously stores moving positions of target portions and reaching time to each of the moving positions as target values, driving means for moving the target portions, and position detecting means for detecting motion positions of the target portions by the driving means, the motion positions detected by the position detecting means are brought into synchronism with target values previously stored, the target values are sequentially read out, each detected motion position and each target value are compared to drive the driving means.

According to this invention, each motion positions detected by the position detecting means are brought into synchronism with each target values previously stored.

A bed driving apparatus of the invention described in claim 74 comprises storing means which previously stores moving positions of target portions and reaching time to each of the moving positions as target values, driving means for moving the target portions, and position detecting means for detecting motion positions of the target portions by the driving means, the motion positions detected by the position detecting means are brought into synchronism with target values previously stored, the target values are sequentially read out, generated target values which are continuous are calculated per unit time which is previously determined by position information between two continuous target positions, the detected motion position and each generated target value are compared to drive the driving means.

According to this invention, the section of the motion target can be brought into synchronization with the generated target position obtained by dividing the motion target section further finely by the constant time interval and thus, optimal moving speed set when it is designed can always be kept.

In a bed driving apparatus of the invention described in claim 75, in the apparatus of claim 73 or 74, when the driving means is started or stopped, voltage to be applied to the driving means is stepwisely changed based on preset arbitrary acceleration variation value.

According to this invention, when the driving means is started or stopped, voltage to be applied to the driving means is stepwisely changed based on preset arbitrary acceleration variation value. Therefore, the acceleration can be controlled without impact.

In a bed driving apparatus of the invention described in claim 76, in the apparatus of claim 73 or 74, when a difference between the target value and a detected moving position exceeds a preset excessive load detection difference value, this state is judged as an excessive load state.

According to this invention, when a difference between the target value and a detected moving position exceeds a preset excessive load detection difference value, this state is judged as an excessive load state. Therefore, it is unnecessary to add another part, and it is possible to prevent the bed from being destroyed in the excessive load state.

In a bed driving apparatus of the invention described in claim 77, in the apparatus of claim 73 or 74, when a difference between the target value and a detected moving position exceeds a preset sandwiching detection difference value, this state is judged as a sandwiching state.

According to this invention, the sandwiching state is judged from deviation between the moving position of the driving means and the actual position in the opposite direction, it is unnecessary to add another part, and it is possible to provide a sandwiching preventing function in the entire case in which there is a possibility that sandwiching constituted by the driving target portion is generated.

### BRIEF DESCRIPTION OF THE DRAWINGS

Fig.1(a)is an explanatory diagram of a basic phantom vertical axis and action around the axis, Fig.1(b) is an explanatory diagram of a phantom longitudinal axis and action around the axis, and Fig.1(c) is an explanatory diagram of a phantom lateral axis and action around the axis;
Fig.2(a) is a phantom horizontal diagram for explaining the action around the vertical axis according to a first embodiment of the present invention, Fig. 2 (b) is a phantom horizontal diagram in which a right shoulder is rising, Fig.2(c) is a phantom horizontal diagram in which the right shoulder is rising, and Fig.2(d) is a phantom horizontal diagram in which the right shoulder is rising;
Fig.3(a) is a phantom horizontal diagram for explaining the action around the longitudinal axis according to the first embodiment of the present invention, Fig.3(b) is a phantom horizontal diagram in which an upper body is turned rightward, Fig.3(c) is a phantom horizontal diagram in which the upper body is turned leftward, and Fig.3(d) is a phantom horizontal diagram in which a lower body is turned rightward;
Fig.4(a) is a phantom horizontal diagram for explaining the action around the lateral axis according to the first embodiment of the present invention, Fig.4(b) is a phantom horizontal diagram in which a right shoulder is rising, Fig.4(c) is a phantom horizontal diagram in which a left side is rising, and Fig.4(d) is a phantom horizontal diagram in which the right and left shoulders are rising;
Fig.5 is a perspective view of a bed according to an embodiment of the invention;
Fig.6 is an exploded perspective view showing a driving structure of the bed;
Fig. 7 is a side view showing one motion state of the driving structure of the bed;
Fig.8 is a side view showing another motion state of the driving structure of the bed;
Fig.9 is a sectional view of an essential portion showing a bottom pipe free joint of the bed;
Fig.10 is a diagram showing swinging locus of the bottom of the bed;
Fig.11 is an exploded perspective view showing a driving structure of a bed according to another embodiment of the invention;
Fig. 12 is a block diagram showing one embodiment of a driving control apparatus of the bed of the invention by means of function realizing means;
Fig.13 is a block diagram showing another embodiment of the driving control apparatus of the bed of the invention by means of function realizing means;
Fig.14 is a block diagram showing another embodiment of the driving control apparatus of the bed of the invention by means of function realizing means;
Fig.15 is a block diagram showing another embodiment of the driving control apparatus of the bed of the invention by means of function realizing means;
Fig.16 is a block diagram showing another embodiment of the driving control apparatus of the bed of the invention by means of function realizing means;
Fig.17 is a block diagram showing another embodiment of the driving control of the bed of the invention;
Fig.18 is a perspective view of a bed having a load sensor corresponding to an embodiment of the invention;
Fig.19 is a block diagram of another embodiment of the invention;
Fig.20 is a block diagram of another embodiment of the invention;
Fig.21 is a conceptual structure diagram showing a state in which a person lies on a bed;
Fig.22 is a block diagram of another embodiment of the invention;
Fig.23 is a conceptual structure diagram showing a state in which a person lies on a bed;
Fig.24 is a block diagram of another embodiment of the invention;
Fig.25 is a block diagram showing another embodiment of the driving control apparatus of the bed of the invention by means of function realizing means;
Fig.26 is a graph showing results of experiment of optimal speed of vertical movement of a back rising bottom;
Fig.27 is a perspective view schematically showing a structure of a bed operating apparatus of an embodiment of the invention;
Fig. 28 is a block diagram showingfunction of the operating apparatus;
Fig.29 is a sectional view of an essential portion of the operating apparatus;
Fig.30 is a circuit diagram schematically showing a structure of the operating apparatus;
Fig.31 is a flowchart showing determining process of operating command of the operating apparatus;
Fig.32 is a graph showing motion state of operating will-association illumination of the bed operating apparatus of the embodiment of the invention;
Fig.33 is a graph showing motion state of daze moderating illumination of the bed operating apparatus of the embodiment of the invention;
Fig.34 is a circuit diagram schematically showing a structure including control means of the driving apparatus of the bed of the embodiment of the invention;
Fig.35 is a graph showing action caused by a continuous driving control method of the driving apparatus of the bed of the embodiment of the invention;
Fig. 36 is a flowchart showing a continuous driving process of the driving apparatus of the bed of the embodiment of the invention;
Fig.37 is a graph showing a principle of a section speed control method in the driving apparatus of the bed of the embodiment of the invention;
Fig.38 is a graph showing motion state at the time of acceleration of shock absorbing driving means in the driving apparatus of the bed of the embodiment of the invention;
Fig.39is a graph showing motion state at the time of deceleration of the shock absorbing driving means in the driving apparatus of the bed of the embodiment of the invention;
Fig.40 is a graph showing a principle of excessive load detecting means in the driving apparatus of the bed of the embodiment of the invention; and
Fig.41 is a graph showing a principle of sandwiching detecting means in the driving apparatus of the bed of the embodiment of the invention.

### BEST MODE FOR CARRYING OUT THE INVENTION

An apparatus and a method for moving a body according to an embodiment of the present invention will be explained with reference to the drawings below.

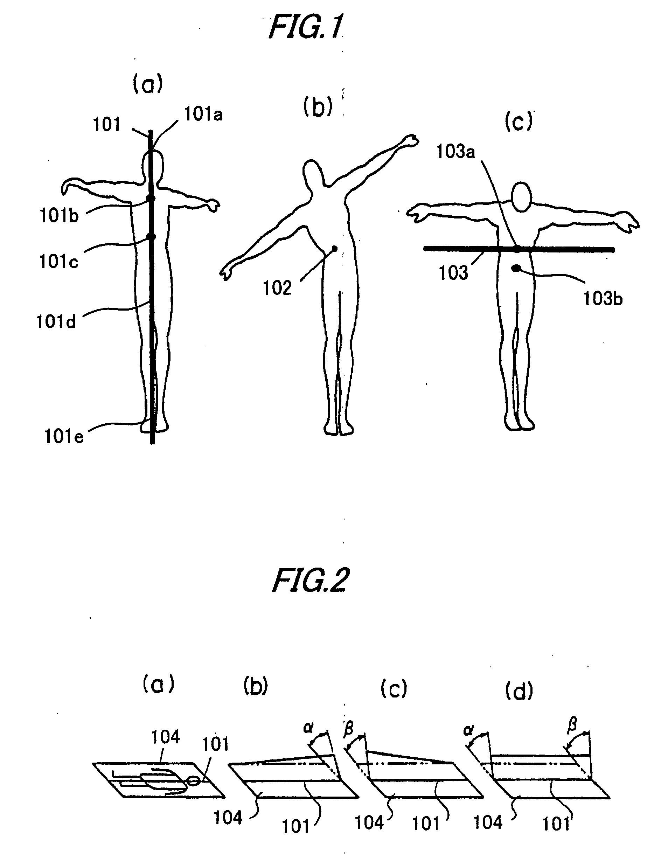

Figs.1 are explanatory diagrams showing a basic phantom axis when the body is allowed to move and action around the phantom axis.

As shown in Fig.1(a), an axis starting from a top center portion 101a of a head of a human body and passing through a chest center portion 101b, an abdomen center portion 101c, a center portion 101d of both legs and reaching a center portion 101e is defined as a vertical axis 101. As shown in Fig.1(b), an axis passing through a laterally naturally bendable portion of the human body near the abdomen center or loin center in the longitudinal direction is defined as a longitudinal axis 102. Further, as shown in Fig.1(c), an axis passing through a center of a longitudinally naturally bendable portion of the human body near an abdomen 103a or a loin 103b is defined as a lateral axis 103. The human body moves variously basically around these three phantom axes 101, 102 and 103.

When a man stands upright, a motion around the vertical axis 101 shown in Fig.1(a) is motion for rotating the body in the horizontal direction with respect to a ground like a turning back motion. This rotation motion includes motion of only the upper body, the abdomen, the loin, or lower body , and includes compound motion of the head and the upper body. Except motion in which the entire body is uniformly rotated, the compound action of the head and the upper body includes twisting motion in generally.

That is, if only the head is rotated, the twisting motion is caused in a neck, and if the upper body is rotated, the twisting motion is caused in abdomen or loin. Thus, the rotation action around the vertical axis 101 can also be called twisting action.

Next, action around the longitudinal axis 102 shown in Fig.1(b) is a rotating action around the longitudinal axis 2 on a vertical plane with respect to a ground such as action for inclining a body for keeping one's lateral balance by spreading both arms during rope-walking for aerobatics. This action includes action of only upper body or lower body, and compound action of both the upper and lower bodies. A position of the longitudinal axis 102 is varied with respect to a subject such as a wall in some cases.

Action around the lateral axis 3 shown in Fig.1(c) is rotating action around the lateral axis 103 such as action of leaning forward and backward on the vertical plane with respect to the ground like the action around the longitudinal axis 102. A position of the lateral axis 103 is varied with respect to the subject such as the wall like the position of the longitudinal axis 102 in some cases.

The present invention improves physical condition and keeps excellent condition by carrying out the action around the three phantom axes 101, 102 and 103 singularly or in combination.

One embodiment of each of the action methods will be explained using Figs.2 to 4.

First, action around the vertical axis 101 will be explained. Figs.2 are explanatory diagrams showing the one embodiment of the action method around the vertical axis.

As shown in Figs.2, a rectangular phantom horizontal plane 104 having space in which a human can lie down is assumed, and it is assumed that a user lies down on this phantom horizontal plane 104. If the user is lying down on his or her back such that portions of the user from his or her head to a center of both legs are placed in the long side direction on a central portion of short sides of the phantom horizontal plane 104, the vertical axis 101 can be assumed on the central portion of the short sides on the phantom horizontal plane 104. Action of the user will be explained using action on the phantom horizontal plane 104 around the vertical axis 101.

That is, a right shoulder side angle of the four angles of the phantom horizontal plane 104 is brought up through an angle α (Fig.2(b)). A right leg side angle of the phantom horizontal plane 104 is brought up through an angle β (Fig.2(c)). A right shoulder side angle of the phantom horizontal plane 104 is brought up through the angle α, and the right leg side angle of the phantom horizontal plane 104 is brought up through the angle of β (Fig.2(d)). Of course, a left shoulder side angle or a left leg side angle of the phantom horizontal plane 104 can similarly be brought up through a certain angle, and four corners of the phantom horizontal plane 104 can be lowered through a certain angle. By moving in this manner, it is possible to variously move the body around the vertical axis 101, and such motion can be expressed concretely.

For example, the right shoulder side angle is brought up through 5° and the left shoulder side angle is lowered through 5°. In this case, the entire phantom horizontal plane 104 is twisted through 5° from the right shoulder toward the left shoulder. Further, the right shoulder side angle is brought up through 10° and the left leg side angle is lowered through 5°. In this case, the entire phantom horizontal plane 104 is inclined and twisted from the right shoulder toward the left shoulder. That is, by determining the positions of the four corners and the concrete rising and lowering angles, various motions can easily be expressed concretely.

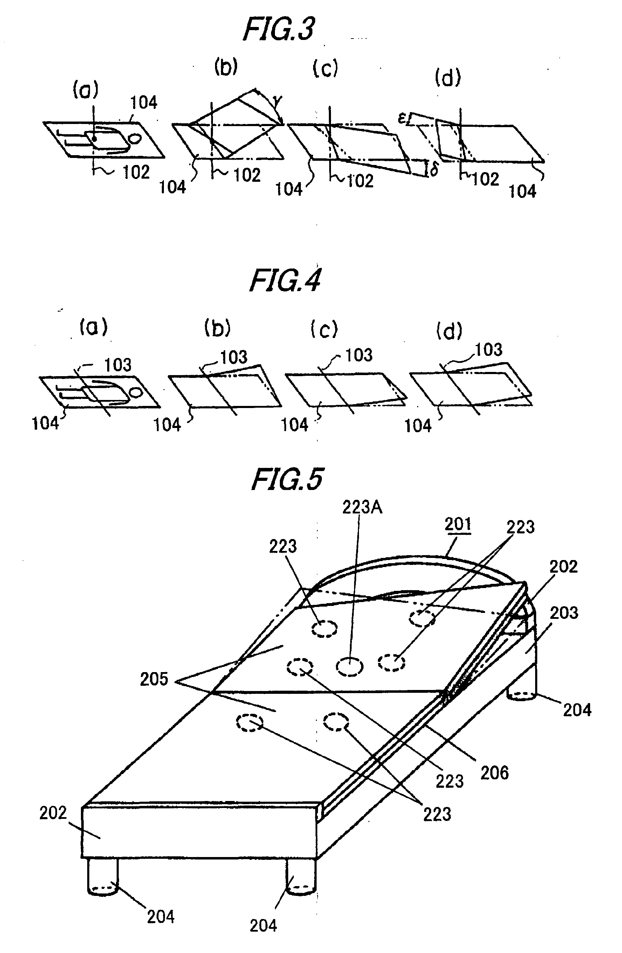

Next, motion around the longitudinal axis 102 will be explained. Fig.3 is an explanatory view showing one embodiment of the moving method around the longitudinal axis. The same explanation as that of the motion around the vertical axis 101 will be omitted.

As shown in Figs.3, it is assumed that the longitudinal axis 102 exists around the center of the bendable abdomen of the man lying down on his or her back. This longitudinal axis 102 stands vertically with respect to the phantom horizontal plane 104. A surface of the phantom horizontal plane 104 on the side of the upper body is moved horizontally around the longitudinal axis 102 toward the right shoulder side through an angle γ (Fig.3(b)). The surface of the phantom horizontal plane 104 on the side of the upper body is moved toward the left shoulder side around the longitudinal axis 102 through an angle of δ (Fig.3(c)). Next, a surface of the phantom horizontal plane 104 on the side of the lower body is moved toward the right leg through an angle of ε (Fig.3(d)). In this manner, the surfaces of the phantom horizontal plane 104 on the side of the upper body and the lower body are moved toward the right shoulder and the left shoulder through certain angles respectively, or the surfaces of the phantom horizontal plane 104 on the side of the upper body and the lower body are moved in the same direction through certain angles respectively. With this, it is possible to variously move the body around the longitudinal axis 102 and to express the motion concretely.

For example, the surface of the phantom horizontal plane 104 on the side of the upper body is moved toward the right shoulder through 10°, and the surface of the phantom horizontal plane 104 on the side of the lower body is moved toward the right leg through 5°. In this case, the phantom horizontal plane 104 is bent rightward into substantially L-shape. Further, the surface of the phantom horizontal plane 104 on the side of the upper body is moved toward the left shoulder through 10°, and the surface of the phantom horizontal plane 104 on the side of the lower body is moved toward the right leg through 5°. In this case, the phantom horizontal plane 104 is bent leftward into slight L-shape and the entire phantom horizontal plane 104 is rotated slightly rightward. That is, by determining the surface on the side of the upper body and the surface on the lower body into certain angles, various motions can easily be expressed concretely.

Next, motion around the lateral axis 103 will be explained. Figs. 4 are explanatory views showing one embodiment of the moving method around the lateral axis. The same explanation as that of the motion around the vertical axis 101 or the longitudinal axis 102 will be omitted.

As shown in Figs.4, it is assumed that the lateral axis 103 exists around the center of the bendable abdomen or loin of the man lying down on his or her back. It is conceived that the lateral axis 103 exists on a position that is slightly deviated toward the lower body with respect to a center of a long side of the phantom horizontal plane 104. If each of the four corners of the phantom horizontal plane 104 is moved vertically alone or in combination, the body can be moved around the lateral axis 103 variously, and the motion can be expressed concretely.

If positions of the head or leg of a human body are not assumed and only the phantom horizontal plane 104 is taken into consideration, the motion around the lateral axis 103 is substantially the same as the motion around the vertical axis 101. That is, the only difference is that the axis exists in the long side direction or in the short side direction of the phantom horizontal plane 104. Therefore, explanation of angle caused by the vertical motion of the concrete four corners will be omitted, but in summary, by determining the positions of the four corners and the concrete vertical motion angle, various motions can be expressed concretely.

Various motions around the three axes were explained above, but these motions around the three axes may be carried out singularly or in combination. The motion may be regularly.

That is, motions around two or three axes, e.g., the vertical axis 101 and the longitudinal axis 102, the longitudinal axis 102 and the lateral axis 103, the lateral axis 103 and the vertical axis 101, or vertical axis 101, the longitudinal axis 102 and the lateral axis 103 may be combined. Further, the single motion or combined motion may carried out regularly by repeating the motion at equal time intervals, e.g., six or ten times per minute. The single motion or combined motion may carried out regularly by repeating the motion at time intervals which are reduced from six to ten times per minute or which are increased from ten to six times per minute. The single motion or combined motion may carried out regularly by repeating the motion regularly at equal angle, e.g., 5° or 10°. The single motion or combined motion may carried out regularly by repeating the motion regularly at equal angle which is gradually increased from 5° to 10° or which is gradually reduced from 10° to 5°. The vertical motion of the right shoulder side angle and the vertical motion of the left leg side angle may be carried out in the same direction or opposite direction repeatedly, the motion from the right shoulder side angle to the left leg side angle may be repeated, or the plurality of motions may be carried out regularly. The single motion or combined motion may be a combination of motion in which the body is moved to a certain angle or position and motion in which a stationary state is maintained for a predetermined time at that position.

It is important that a user can set a magnitude or repetition of the above-described motion in accordance with his or her own body, and the user can select the magnitude or repetition from a previously registered pattern and setting.

If the user sets, selects and executes the motion method or pattern which is suitable for the body of the user, it is possible to bring a bad portion of the body (neck, leg, foot, loin or the like) into excellent condition, to maintain the user's body excellently when the user's body is in good condition, and to spend healthy and comfortable life.

The motion method or pattern set or selected by the user is stored individually, and it is possible to maintain relaxed attitude which is suitable for the body of the user before the user sleeps, and to sleep comfortably.

By executing the motion method or pattern set or selected for each of the users, it is possible to adjust the attitude for correcting the distortion or unbalanced portion of the body, or to fix the motion suitably for the distortion or unbalanced portion of the body, thereby keeping the relaxed attitude.

An embodiment in which the above-described phantom horizontal plane 104 is applied to a bed will be explained.

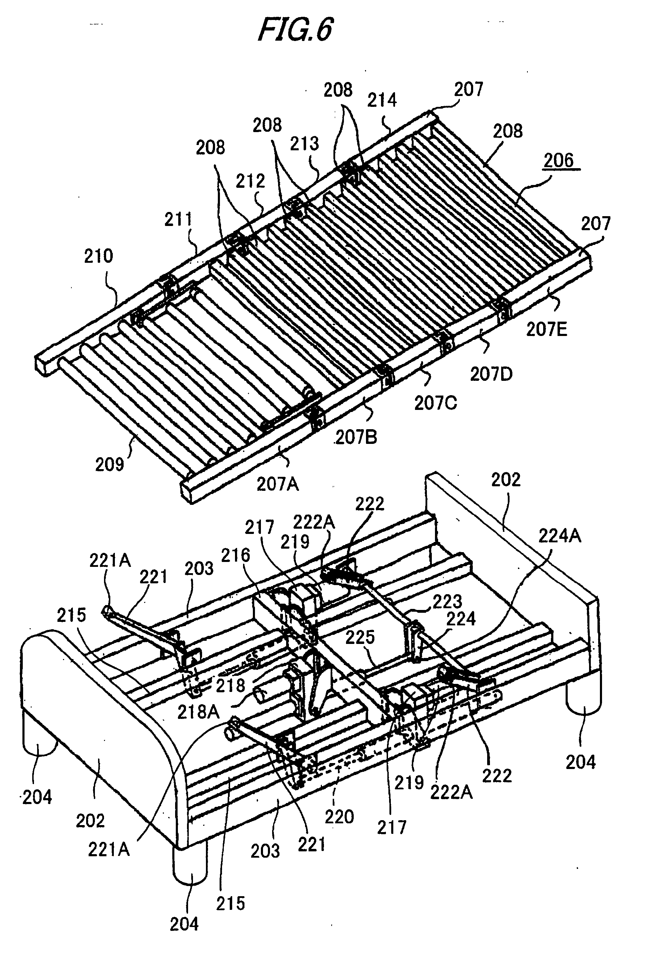

First, the entire structure of the bed of the present embodiment will be explained using Fig.5.

Fig.5 is a perspective view of the bed of the embodiment of the present invention.

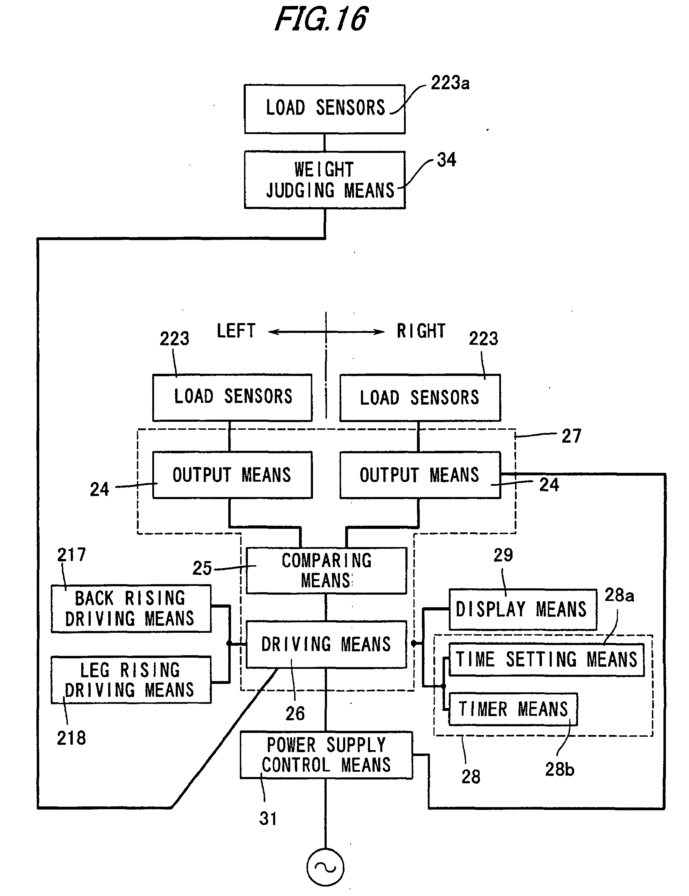

As shown in Fig.5, the bed 201 of the embodiment comprises a pair of legs 204, each the pair having two legs, a pair of bases 202 fixed to the legs 204, rack frames 203 for connecting both the bases 202, and a bottom 206 placed on the rack frames 203 for partially moving mats 205 placed on upper surface of the rack frames 203 in the various angle. The mats 205 are provided with a plurality of load sensors 223. A load sensor 223 will be explained in an embodiment shown in Fig.16.

Next, a driving structure of the bed of this embodiment will be explained using Figs.6 to 10.

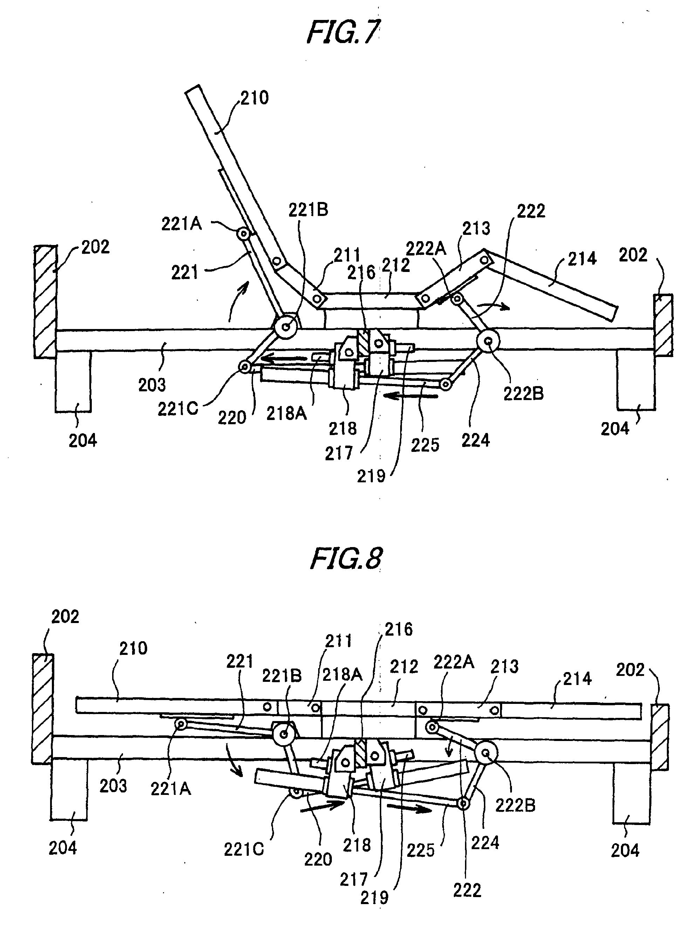

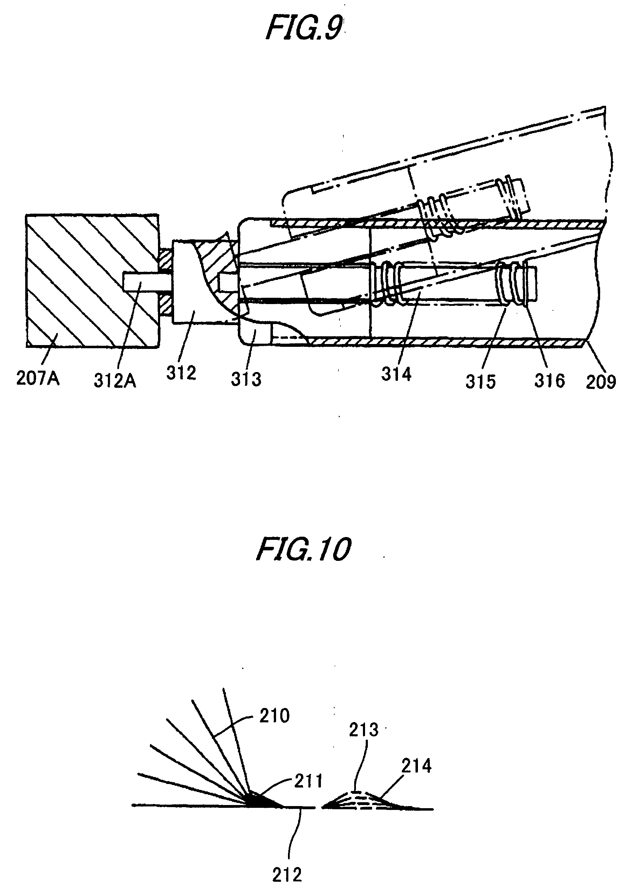

Fig. 6 is an exploded perspective view showing the driving structure of the bed, Fig.7 is a side view showing one motion state of the driving structure of the bed, Fig.8 is a side view showing another motion state of the driving structure of the bed, Fig.9 is a sectional view of an essential portion showing a bottom pipe free joint of the bed, and Fig.10 is a diagram showing swinging locus of the bottom of the bed.

As shown in Fig.6, the pair of bases 202 and the pair of rack frames 203 constitute a rectangular frame body, and a rectangular bottom 206 on which the mattress or the like is placed is mounted on the frame body.

The bottom 206 comprises left and right side frames 207 constituting a frame on the side of the long sides of the rectangular frame body, as well as frame members 208 and bottom pipes 209 connecting the side frames 207 with each other. Each of the pair of side frames 207 is divided into five pieces, i.e., side frames 207A, 207B, 207C, 207D and 207E of different length. Thus, the bottom 206 is divided into aback rising bottoms 210, 211, a fixing bottom 212, a leg rising bottoms 213 and 214 from the head toward the leg. The back rising bottom 210 and the fixing bottom 212; the back rising bottom 211 and the fixing bottom 212; the fixing bottom 212 and the leg rising bottom 213; the leg rising bottom 213 and the leg rising bottom 214 are rotatably connected to each other by hinges. Since the bottoms 210, 211, 212, 213 and 214 are rotatably connected to each other by the hinges, height and inclination of the bottom surfaces of the bottoms 210, 211 and 213 can be changed with respect to the fixing bottom 212.

In the back rising bottom 210, a plurality of bottom pipes 209 are connected between the left and right side frames 207A by means of a free joint 312 shown in Fig.9. By connecting the bottom pipes 209 to the side frames 207A and 207E by the free joint 312, a twisting mechanism can be formed.

Here, turning connection of the bottom pipe 206 will be explained using Fig.9.

As shown in Fig.9, the free joint 312 is provided at its one end with a projection 312A to be mounted to the side frame 207A, and at its other end with a guide pin 314. A slide block 313 is slidably and turnably provided on the guide pin 314. A portion of the slide block 313 is inserted and fixed in the bottom pipe 209. A compression spring 315 is mounted to the guide pin 314. One end of the compression spring 315 is fixed to an end of the guide pin 314 by means of a stopper 316. The other end of the compression spring 315 abuts against the slide block 313. The slide block 313 is biased toward the free joint 312 by means of the compression spring 315.

On the other hand, as shown in Fig. 6, the back rising bottom 211 fixes the left and right side frames 207B by means of the plurality of frame members 208. The fixing bottom 212 fixes left and right side frames 207C by means of the plurality of frame members 208. The leg rising bottom 213 fixes the left and right side frames 207D by means of the plurality of frame members 208.

In the rack frame 203, two base frames 215 which are parallel to the long side are fixed to the bases 202 at a predetermined distance from each other, and a base frame 216 is fixed to the rack frames 203 at a location closer to the leg with respect to a central portion of the base frame 215. Two back rising driving means 217 for operating the bottoms 206 are provided on the left and right base frames 215 closer to the leg. Leg rising driving means 218 are provided on the base frame 216. The back rising driving means 217 comprises a motor 219, a rack (not shown) and a pinion (not shown) for converting the rotation motion of the motor 219 to the reciprocating motion, a back rising arm 221, and a rod 220 for transmitting driving power to the back rising arm 221. The two back rising arms 221 are of substantially L-shape, and are provided at left and right positions closer to a head of the base frame 215. Each of the back rising arms 221 is turnably supported by a distortion angle portion 221B so that the arm 221 swings in parallel to the base frame 215. One end 221C of the back rising arm 221 is turnably supported by a rod 220, and a roller 221A is provided on the other end of the back rising arm 221. The roller 221A of the back rising arm 221 slidably abuts against a lower surface of each of the left and right side frames 207A of the back rising bottom 210.

The two leg rising arms 222 are provided in parallel to each other on left and right positions closer to the leg of the base frames 215. One end 222B of each of the leg rising arms 222 is turnably supported by a connection member 223 so that the leg rising arm 222 swings in parallel to the base frame 215. The one end 224A of the arm 224 provided on the connection member 223 is turnably supported by the rod 225. The leg rising arm 222 is provided at its other end with a roller 222A. The roller 222A of the leg rising arm 222 slidably abuts against a lower surface of the left and right side frames 207D of the leg rising bottom 213. Here, leg rising driving means 218 comprises a rack (not shown) and a pinion (not shown) which convert the rotation motion of the motor 218A into reciprocating motion, the leg rising arm 222, and the rod 225 which transmits the driving power to the leg rising arm 222. The leg rising driving means 218 transmits the driving power to the arm 224 through the rod 225.

One embodiment of motion of the above structure will be explained below.

This embodiment shows motion for 1/f swinging the back rising bottom 210.

First, leaning angle, swinging angle and driving time of the back rising bottom 210 are designated.

When the driving is started, the motor 219 of the back rising driving means 217 is rotated, the rod 220 is moved horizontally, the other end having the roller 221A of the back rising arm 221 is moved upward of the bed. By the motion of the back rising arm 221, the back rising bottom 211 is inclined around a connection with the fixed bottom 212, the back rising bottom 210 is moved upward of the bed and inclined. When the back rising bottom 210 is inclined through a predetermined angle, the motion of the back rising driving means 217 is stopped.

Next, the 1/f swinging motion is designated. The swinging motion is carried out by repeatedly rotating motors 219 of left and right two back rising driving means 217 in the opposite directions in normal and reverse direction. That is, by rotating the motors 219 in the opposite direction, the left and right rods 220 moves in the opposite directions, the left and right two side frames 207A moves in the opposite directions. Therefore, a surface of the back rising bottom 210 is twisted, and the swinging motion is carried out.

At that time, a variation amount of expansion and contraction of straight distance between left and right side frames 207A is absorbed by universal joint 312 of the bottom pipe 209. If a set driving time is elapsed, the swinging motion is stopped. A motion for returning the inclination of the back rising bottom 210 to a horizontal position when the operation is stopped can be realized by setting the motion previously.

The locus as viewed from a side of the bottom is shown in Fig.10.

As shown in Fig.10, the back rising bottom 211 has one end on the side of the fixed bottom 212 and the other end. The other end vertically moves around the one end. One end of the back rising bottom 210 on the side of the back rising bottom 211 vertically moves together with the back rising bottom 211, and the other end further vertically moves around the one end thereof. The inclination angle of the back rising bottom 210 increases as the inclination angle of the back rising bottom 211 increases.

On the other hand, the leg rising bottom 213 has one end on the side of the fixed bottom 212, and the other end thereof moves vertically around the one end of the leg rising bottom 213. One end of the leg rising bottom 214 on the side of the leg rising bottom 213 vertically moves together with the leg rising bottom 214. A connected portion between the leg rising bottom 213 and the leg rising bottom 214 bends knees.

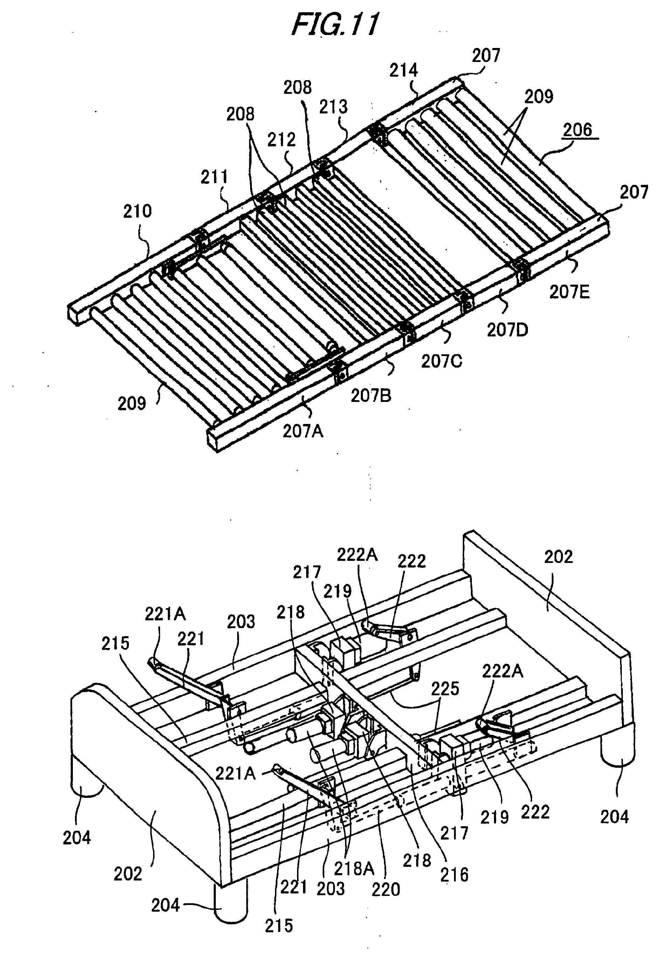

Next, a bed driving structure of another embodiment will be explained using Fig.11.

Fig.11 is an exploded perspective view showing the bed driving structure. Members having the same functions as those explained in the embodiment shown in Figs.5 to 9 are designated with the same numbers, and explanation thereof is omitted. A portion of this embodiment corresponding to Fig.6 is illustrated, other members which are not illustrated in Fig.11 are the same as those in the embodiment shown in Fig.5 to 9 and thus, these members are not illustrated.

In the embodiment shown in Fig.11, two leg rising driving means 218 are provided, and these two leg rising driving means 218 can independently move a pair of leg rising arms 222. A plurality of bottom pipes 209 of the leg rising bottom 214 are connected to each other by universal joint 312 shown in Fig.9 between the left and right side frames 207E. The structure and motion of the universal joint 312 is the same as those shown in Fig.9 and thus, explanation thereof is omitted.

Two leg rising arms 222 are provided at left and right sides in parallel to each other at positions of the base frames 215 closer to legs. One end 222B of the leg rising arm 222 is turnably supported by the rod 225 such that the one end swings in parallel to the base frame 215. The other end of the leg rising arms 222 slidably abut against lower surface of the left and right side frames 207E of the leg rising bottom 214. The leg rising driving means 218 has basically the same structure as that of the back rising driving means 217, and the two leg rising driving means 218 are provided at center positions of the base frame 216. The leg rising driving means 218 transmits driving force to the arm 224 through the rod 225.

An embodiment of the action of the structure will be explained below.

This embodiment shows action for allowing the leg rising bottom 214 to 1/f swing.

First, leaning angle, swinging angle and driving time of the leg rising bottom 214 are designated.

When the driving is started, the motor 218A of the leg rising driving means 218 is rotated, the rod 225 is moved horizontally, the other end having the roller 222A of the leg rising arm 222 is moved upward of the bed. By the motion of the leg rising arm 222, the leg rising bottom 213 is inclined around a connection with the fixed bottom 212, the leg rising bottom 214 is moved upward of the bed and inclined. When the leg rising bottom 214 is inclined through a predetermined angle, the motion of the leg rising driving means 218 is stopped.

Next, the 1/f swinging motion is designated. The swinging motion is carried out by repeatedly rotating motors 218A of left and right two leg rising driving means 218 in the opposite directions in normal and reverse direction. That is, by rotating the motors 218A in the opposite direction, the left and right rods 225 moves in the opposite directions, the left and right two side frames 207E moves in the opposite directions. Therefore, a surface of the leg rising bottom 214 is twisted, and the swinging motion is carried out.

At that time, a variation amount of expansion and contraction of straight distance between left and right side frames 207E is absorbed by universal joint 312 of the bottom pipe 209. If a set driving time is elapsed, the swinging motion is stopped. A motion for returning the inclination of the leg rising bottom 214 to a horizontal position when the operation is stopped can be realized by setting the motion previously.

It is also effective to combine this swinging motion with the swinging motion of the back rising bottom 210, and to set these in staggered twisting directions. If the swinging motion of the back rising bottom 210 and the swinging motion of the leg rising bottom 214 are combined and these are set in the staggered twisting directions, even if the swinging twisting amounts of them are reduced, large twisting amount can be obtained.

The bottom 206 of the bed 201 explained in the above embodiment allows the body to move around three axes of the vertical axis 101, the longitudinal axis 102 and the lateral axis 103 explained in Figs.1 to 4. It is preferable operate the motion of the bottom, and to display the motion to be obtained as the result of the operated motion by means of operating means such as a remote control unit. BY displaying the motion, it is possible to operate the motion of the bed 201 while confirming the motion of the bed 201 visually.

In the above structure, a user can set or select the motion suitable for the user's body while lying down. Further, since the user can set a bed surface suitable for the user, the user can sleep in relaxed and comfort posture.

It is possible to adjust distortion and unbalanced portion of the body utilizing various motions. For example, if the user' s right shoulder is distorted, in order to correct the distortion, the body can be adjusted by bringing the right shoulder through a certain angle and bringing the left shoulder down for a constant time. On the other hand, it is possible to obtain relaxed posture by setting the inclination angle in accordance with the distortion or unbalanced portion of the user's body.

With this, it is possible to bring bad body portion into excellent condition, to keep the good body condition as long as possible, and to spend healthy and comfortable life.

Next, a bed driving control apparatus will be explained.

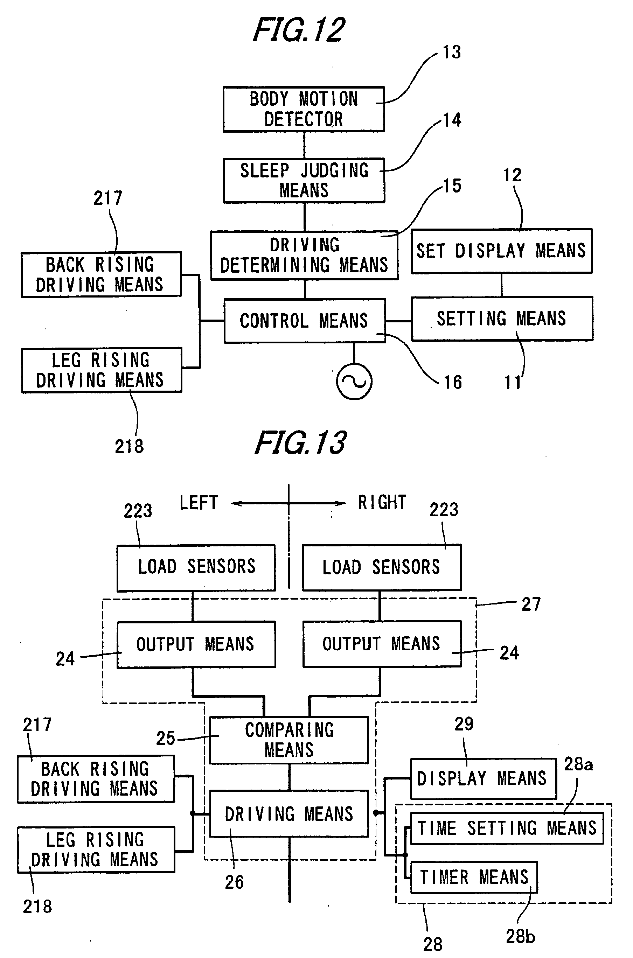

Fig.12 is a block diagram showing an embodiment of the bed driving control apparatus by function realizing means.

This embodiment comprises a body motion detector 3 for detecting motion of the body on the bed 201 provided on the back rising bottom 210, asleep judging means 14 for judging the sleeping state from the detection of the body motion detector 3, driving determining means 15 for determining stop and start of the back rising driving means 217 and the leg rising driving means 218, and a control means 16 for stopping and starting the back rising driving means 217 and the leg rising driving means 218.

The setting means 11 set moving range and moving method of the back rising driving means 217 and the leg rising driving means 218. Here, the moving range is a range of inclination angle, twisting angle, rising height and the like, and the moving method includes 1/f swinging motion, repeating of individual motions, combination of individual motion, rules of motion and the like. The setting means 11 can select or set power level of the motion and pitch, select or set motion start time, completion time or motion time, change or cancel the bottom posture, and set the timer means or the like. In accordance with the moving range and moving method set by the setting means 11, signals are output to the back rising driving means 217 and the leg rising driving means 218 from the control means 16. The contents set by the setting means 11 are displayed by the set display means 12, and can be visually checked. It is preferable that the contents set by the setting means 11 are stored so that the contents are not erased even if power supply is cut as long as the contents are not changed or erased intentionally.

During human' s sleep, "NON-REM sleep" and "REM sleep" which mainly gives respite are alternately repeated. A set (about 90 minutes) of the "NON-REM sleep" and the "REM sleep" are repeated about four times. Tossing and turning are prone to be caused before and after the "REM sleep", and people can wake up in best condition after the last "REM sleep" is completed. The sleep judging means 14 uses body motion sensed by a body motion detector 13 as an input signal, judges the last "REM sleep" in the cycle and its terminal time point, and output a signal to the driving determining means 15. The driving determining means 15 receives the signal from the sleep judging means 14, and output a signal to the control means 16 after a predetermined time. If the control means 16 receives the signal from the driving determining means 15, the control means 16 outputs a driving signal which is previously set in the setting means 11, and starts the motion of the back rising driving means 217 and the leg rising driving means 218.

Fig.13 is a block diagram showing another embodiment of the bed driving control apparatus with the function, realizing means. Structures having the same functions as those of the previous embodiment are designated with the same numbers, and explanation thereof is omitted. The same can be applied hereinafter.

This embodiment comprises a plurality of load sensors 223, output means 24 for outputting a detected load signal, comparing means 25 for comparing an output signal value from the output means 24, and driving means 26 for controlling motion of the twisting mechanism means based on a comparing signal from the comparing means 25. The control means 27 is comprised of the output means, the comparing means and the driving means. The control means 27 is connected with the back rising driving means 217 and the leg rising driving means 218. Connected to the control means 27 are setting means 28 having time setting means 28a and timer means 28b for canceling the bottom posture and like, and display means 29 for displaying set contents and motion state of the setting means 28, and detector measuring state and the like.

A plurality of load sensors 223 are disposed on left and right sides symmetrically with respect to a center line of lying up body inside amat 205 as shown in Fig. 5, and disposed at locations corresponding to shoulder, loin and calf. The load sensors 223 uses load cell or distortion resistant body, and is embedded in an urethane sponge fitted in the mat 205, or is fixed on an upper surface of the mat 205 or fixed on a back side of a bed sheet. The resistant body of the load sensors 223 may use a sheet-like load sensor disposed on a surface thereof.

In the above structure, if the load sensors 223 detects a body on the bed, the load sensors 223 transmits resistant values which are inversely proportional to applied pressure to the output means 24 as load signals. The output means 24 outputs the load signal as output voltage, the comparing means 25 compares the output voltage for each load sensors 223 disposed on the left and right sides symmetrically, and outputs the voltage as load power level distribution state. Upon reception of the signal, the driving means 26 drives the left back rising arm 221 or the right shoulder rising arm 221 by means of the back rising driving means 217, and the back rising bottom 210 is inclined through a designated angle. If the inclination angle of the back rising bottom 210 is determined, the load state of the body in this state is detected again by the load sensors 223. The output means 24 outputs a signal from the load sensors 223 as a load signal, and the driving means 26 repeatedly finely adjust the back rising bottom 210 until the load distribution becomes uniform on the left and right sides. If the load distribution becomes uniform, i.e., the bed user assumes the relaxed posture. the driving means 26 is stopped. The leg rising bottom 214 also controls in the same manner as the back rising bottom 210 so that uniform state can be secured. By controlling the body pressure distribution uniformly, relaxed posture can be obtained.

If the bed user sets average time required to sleep by the time setting means 28a of the setting means 28, the back rising bottom 210 measures elapsed time in the twisted state, and when the output value from the load sensors 223 is not changed within the set time, the back rising bottom 210 judges that the bed user is in a sleeping state, releases the twist, and returns the bottom into a horizontal state or initially set state automatically. Further, it is possible to return the bottom into the horizontal state or initially set state after a predetermined time irrespective of output value from the load sensors 223 by using the timer means 28b. People can not sleep deeply without tossing and turning motion in the sleeping state, and if the bottom surface becomes horizontally automatically, people can sleep deeply.

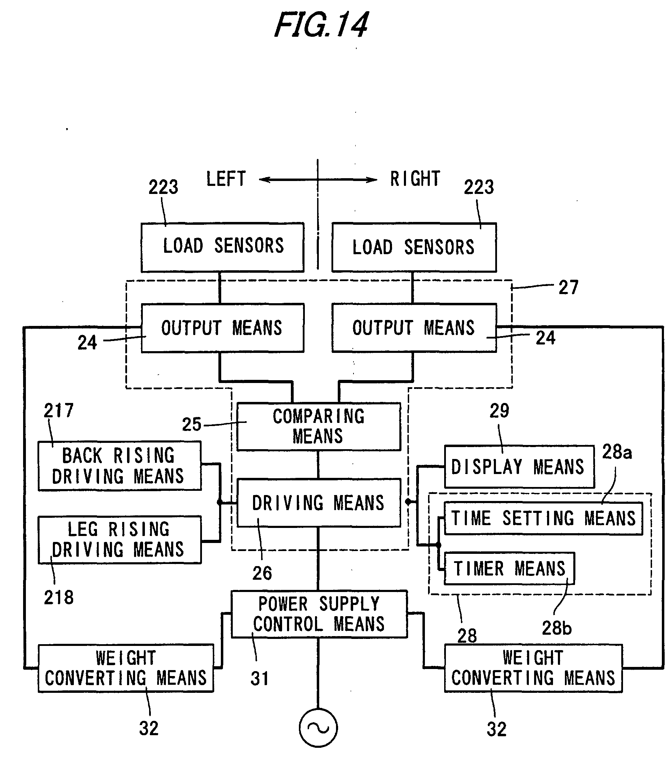

Fig.14 is a block diagram showing another embodiment of the bed driving control apparatus with function realizing means.

In this embodiment, in addition to the structure of the embodiment shown in Fig.13, a power supply control means 31 is connected to the driving means 26, and a weight converting means 32 for converting a load signal to weight is provided between the output means 24 and the power supply control means 31.

In this embodiment, if a user comes on the bed, a load signal from the output means 24 is output from output voltage, an increased weight signal is output from the weight converting means 32, and the power supply control means 31 turns on the driving power supply. If no person is on the bed, the weight signal is returned to a predetermined value and thus, the power supply is turned off. In this manner, if the user exists on the bed, the power supply is turned on, and does not exist, the power supply is turned off and thus, which provides excellent safety and energy saving.

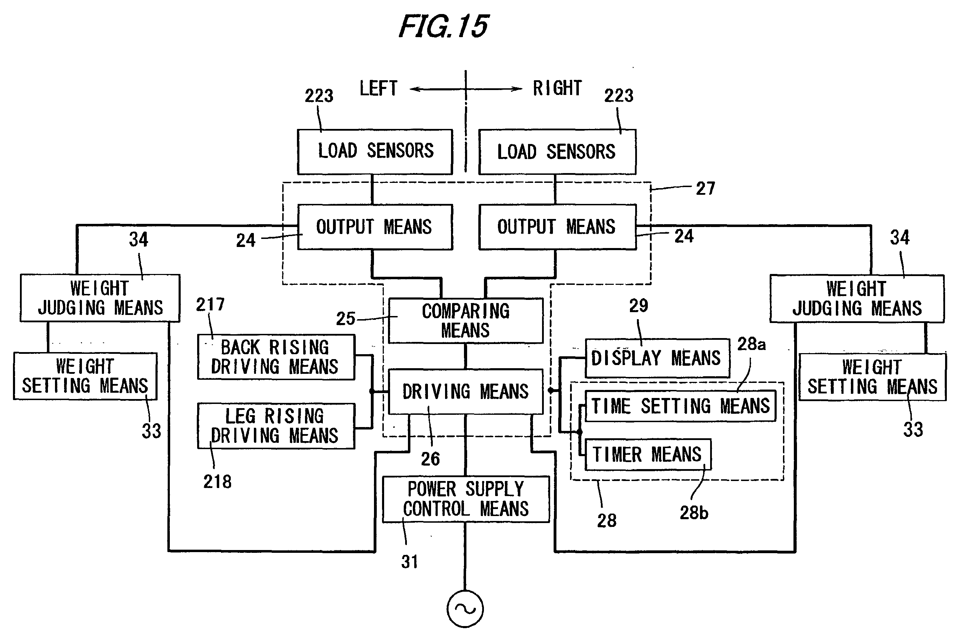

Fig.15 is a block diagram showing another embodiment of the bed driving control apparatus with function realizing means.

In this embodiment, in addition to the structure of the embodiment shown in Fig.13, weight setting means 33 and weight judging means 34 are provided between the output means 24 and the driving means 26.

In this embodiment, weight of the bed user is previously detected or input weight is stored in weight setting means 33. The weight judging means 34 compares weight detected by the load sensors 223 and output from the output means 24 and weight set in the weight setting means 33, and judges. As a result of the judgement of the weight judging means 34, if the weight is lower than or different from the set value, the driving means 26 is locked and is not driven. When the bed user does not exists, even if a baby or pet comes on the bed, safety is secured.

Fig.16 is a block diagram showing another embodiment of the bed driving control apparatus with function realizing means.

In this embodiment, in addition to the structure of the embodiment shown in Fig.13, a load sensor 223a is provided in the mat 5 in a region against which a sacrum of a body abuts, and the load sensor 223a is provided with weight judging means 34 which judges weight based on a signal detected by the load sensor 223a. If the load sensor 223a found weight equal to or lower than a preset value, the load sensor 223a judged that the bed user is lying on his or her back. That is, since the sleeping body on the mat is in a posture in which the tender abdomen comes down, it is judged that no pressure is applied to the load sensor 223a. If the load sensor 223a found the weight equal to or lower than the preset value, the driving means 26 is locked so that it is not driven. The upward twisting motion while lying on his or her back may damage important portion such as backbone, and this embodiment can provide safety means.

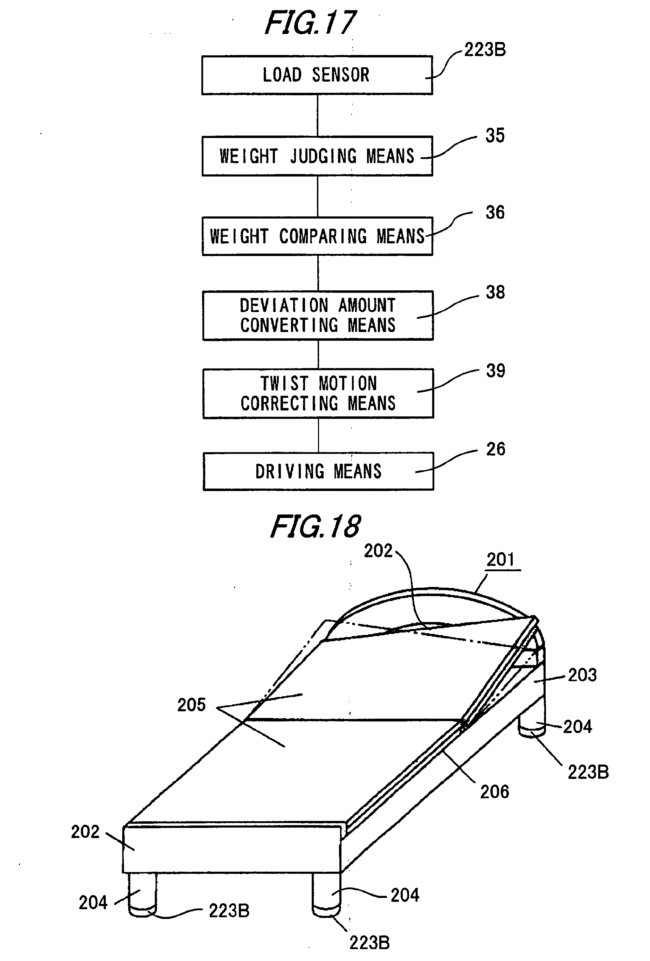

Fig.17 is a block diagram of another embodiment of the bed driving control, and Fig.18 is a perspective view of a bed having a load sensor corresponding to this embodiment.

First, as shown in Fig.18, the bed of the embodiment is provided with load sensors 223B using load cells between a floor and a legs 204 supporting four corners of the rack frames 203 on the floor.

As shown in Fig.17, the bed of the embodiment comprises weight judging means 35 at four locations based on a load signal detected by the load sensor 223B, comparing means 36 comparing weights at four locations judged by the weight judging means 35, deviation amount converting means 38 for converting the compared weight difference into a deviation amount with respect to a center of the bed of a center line in a height direction of the bed user, twisting motion correcting means 39 based on the deviation amount, and driving means 26 for twisting based on the twist motion corrected by the twisting motion correcting means 39.

In this embodiment, weights at the four locations are compared, and if the weight differences are the same, it is judged that a person exists on a central portion of the bed, and the bed is twisted, and if different, the weight difference is converted to the deviation amount of the body position from a center of the bed, and larger weight is reduced such that the twisting driving amount is corrected form the deviation amount. According to this embodiment, wherever the bed user is sleeping on the bed, it is possible to adjust the twist motion in accordance with the body position.

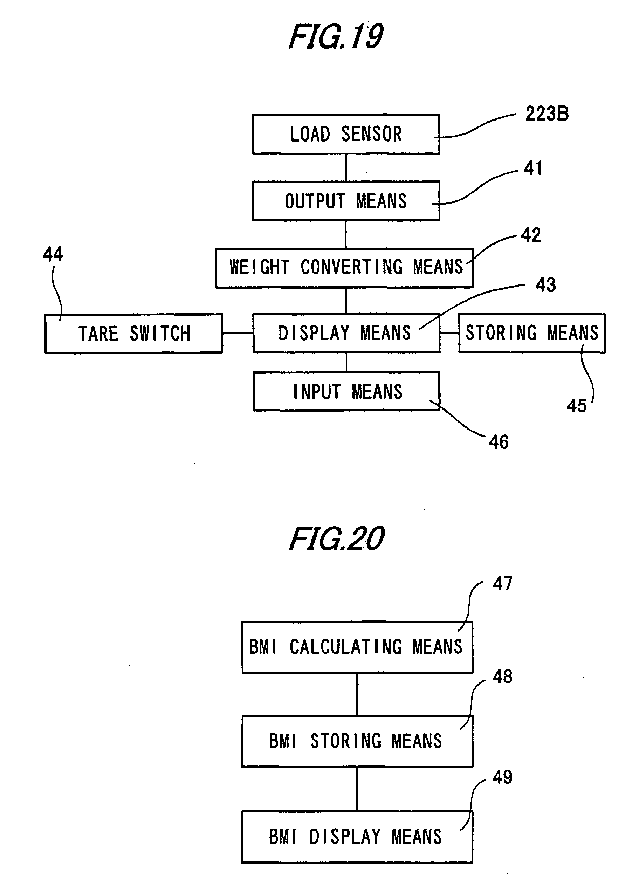

Fig.19 is a block diagram of another embodiment.

As shown in Fig.18, the bed of the present invention is provided with load sensors 223B between the between a floor and a legs 204 supporting four corners of the rack frames 203 on the floor.

As shown in Fig.19, the bed of the embodiment comprises load sensors 223B for detecting load applied to the bed, output means 41 for sending the detected load signal, weight converting means 42 for converting the signal sent from the output means 41 to a weight, display means 43 for displaying the converted weight, a tare switch 44 for previously tarring the load of the bed itself before the user's weight is applied, storing means 45 for storing measured daily weight and measuring date and time, and input means 46 capable of displaying the weight of the measuring date and time if the measuring date and time is input.

According to this embodiment, if the user is sleeping or sitting on the bed and a load is applied to the bed, the load is applied to the load sensors 223B disposed on the legs 204 at the four corners. The loads applied to the load sensors 223B at the four corners are input to the weight converting means 42 through the output means 41. The voltage input to the weight converting means 42 is converted into weight, and the added weight is displayed by the display means 42 as a load applied to the bed. Therefore, according to the embodiment, it is possible to find the user's weight only by lying on the bed, and to judge the health condition.

Since a load of the bed itself is applied before the user's load is applied, it is preferable to subtract the bed's weight previously by the tare switch 44 before the user's weight is measured, and to display the user's weight. It is also possible to detect the reduction of load when the user comes down from the bed and display the same as the weight.

According to this embodiment, if the measuring date and time is input by the input means 46, the weight of the measuring date and time stored in the storing means 45 can be displayed. If the display means is provided with calculating means, it is possible to calculate a graph or weight variation rate of the weight transition during an input designated period and to display the same. By storing the measurement result in this manner, index for judging the health condition can be obtained.

It is preferable to use the function explained in this embodiment together with functions of another embodiment explained above.

Fig.20 is a block diagram of another embodiment.

This embodiment comprises BMI calculating means 47 for calculating fat judging index, BMI storing means 48 for storing BMI calculated by the BMI calculating means 47, and BMI display means 49 for displaying the BMI which was stored in the BMI storing means 48.

According to this embodiment, by inputting the weight measured in each of the embodiments and height separately measured or input, it is possible to calculate the fat judging index, and to obtain data for the health management.

It is preferable to use the function explained in this embodiment together with functions of another embodiment explained above.

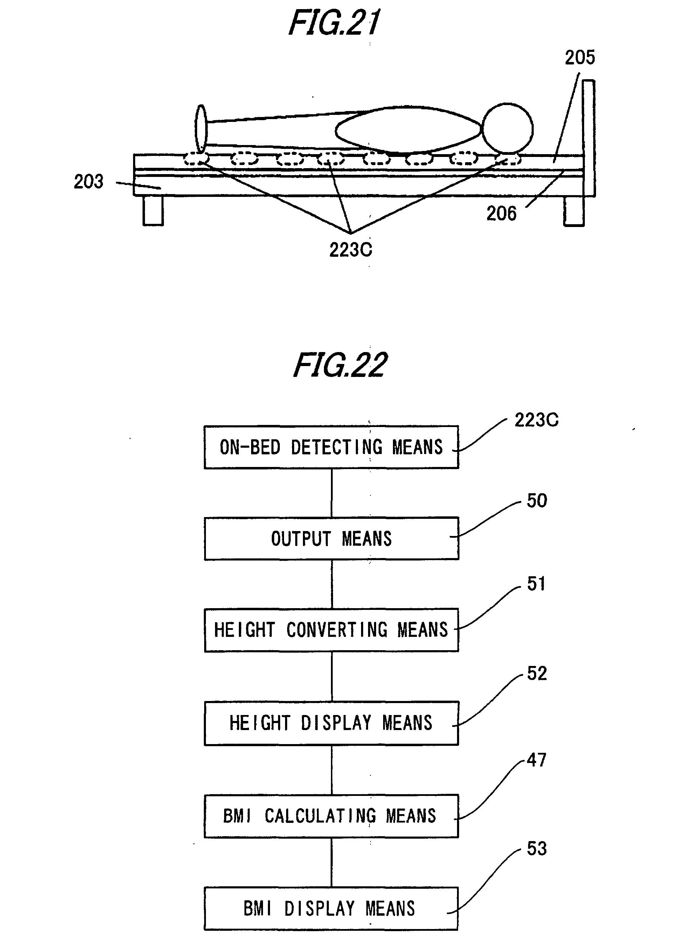

Figs.21 and 22 show another embodiment. Fig.21 is a conceptional structure diagram showing a person lying on a bed, and Fig.22 is a block diagram.

First, as shown in Fig.21, a plurality of load sensors 223c are disposed on a center line of a short side in a direction of a long side in a mat 205 placed on a bottom 206. The load sensor 223c detects weight applied on the mat 205. If a person lies on the bed, the load sensors 223c detect the weight, and output varying resistance value from output means 50 as voltage. Height converting means 51 converts the output voltage into a signal indicative of the height.

According to the embodiment, it is possible to measure the height on the bed. The height measured in this manner can be utilized for the BMI calculating means 47 of the above embodiment. Therefore, if the detecting means 223C of this embodiment is used and the height converting means 51 is provided, it is possible to measure the weight and height at the same time only by lying on the bed, and to find the BMI, and this can be used for judging the health condition.

It is preferable to use the function explained in this embodiment together with functions of another embodiment explained above.

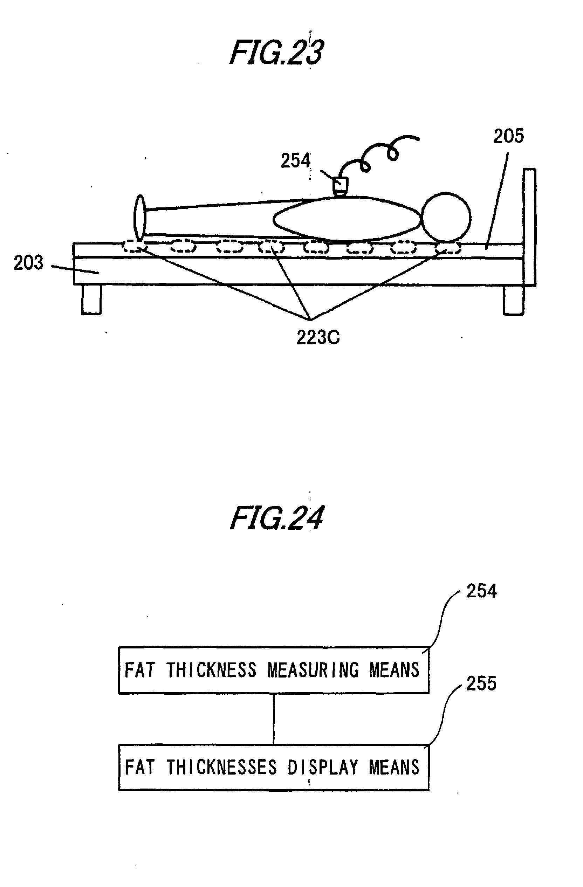

Figs.23 and 24 show another embodiment. Fig.23 is a conceptional structure diagram showing a person lying on a bed, and Fig.24 is a block diagram.

In this embodiment, in addition to the embodiment shown in Fig.21, grease thickness measuring means 254 is provided. This embodiment includes grease thickness display means 255 for displaying grease thickness measured by the grease thickness measuring means 254.

According to this embodiment, it is possible to obtain more data for the health management because the grease thickness display means is also provided.

It is preferable to use the function explained in this embodiment together with functions of another embodiment explained above.

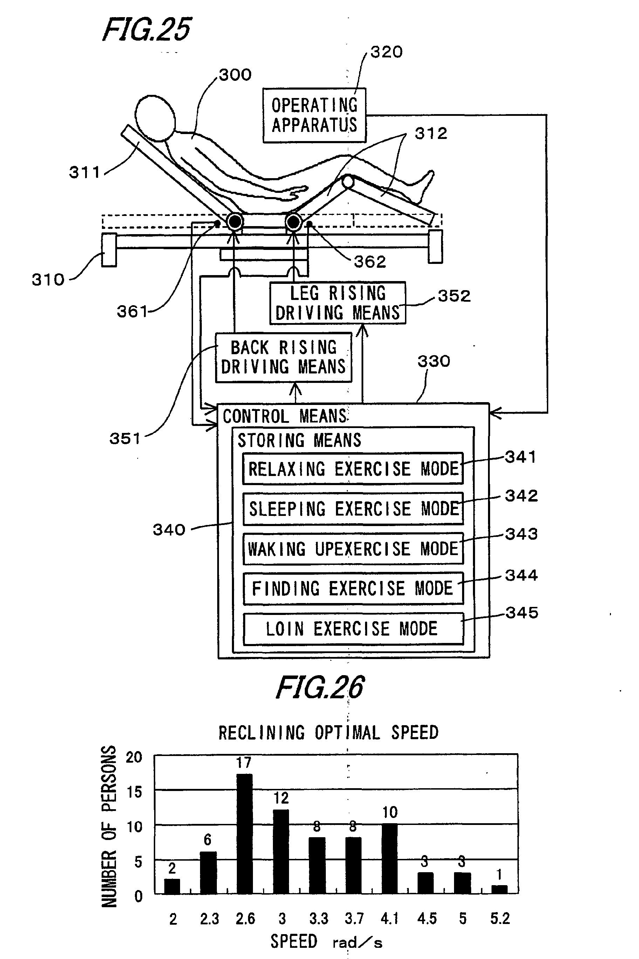

Figs.25 to 51 show another embodiment.

Fig.25 is a block diagram showing a bed driving apparatus with function realizing means.

A bed 310 includes a back rising bottom 311 for supporting a back of a body 300, and a leg rising bottom 312 for supporting legs of the body 300. Here, the back rising bottom 311 is constituted by one member, the angle of the back rising bottom 311 can be changed around its one end, and the back rising bottom 311 can be twisted so that the height of the other end is different in the widthwise direction. On the other hand, the leg rising bottom 312 is constituted by two members, the leg rising bottom 312 is turned around one end of one of the members, the other end of the one member and one end of the other member are turnably connected to each other, and an inclination angle between the two members is increased as an angle of a slant thereof is increased with respect to the horizontal plane. That is, by increasing the inclination angle between the two members, the leg rising bottom 312 is operated to bend knees of the body 300.

The back rising bottom 311 and the leg rising bottom 312 are operated by an operating apparatus 320. The operating contents by the operating apparatus 320 are sent to control means 330. The operating contents are instructed by the operating apparatus 320 are partially stored in storing means 340 previously. Previously stored in the storing means 340 are a relaxing exercise mode 341, a sleeping exercise mode 342, a waking up exercise mode 343, a finding exercise mode 344, a loin exercise mode 345. Other motion patterns are also stored in the storing means 340. The exercise modes and motion patterns stored in the storing means 340 are read out from the control means 330 by instructions of the operating apparatus 320 or detection signals of the load sensor and the like.

The back rising driving means 351 is operated based on an output signal from the control means 330, and allows the back rising bottom 311 to twist or change its inclination angle. The leg rising driving means 352 is operated based on an output signal from the control means 330, and allows the leg rising bottom 312 to twist or change its inclination angle.

Position detecting means 361 detects motion position of the back rising bottom 311, and outputs a position signal to the control means 330. Position detecting means 362 detects motion position of the legrising bottom 312, and outputs a position signal to the control means 330.

The exercise modes previously stored in the storing means 340 will be explained.

In the relaxing exercise mode 341, twist motion to be given to the back rising bottom 311 is carriedout twice or less/second, the back rising bottom 311 is vertically moved in a predetermined angle range with respect to the horizontal plane while being twisted, and the twist motion and the vertical motion are carried out in predetermined rhythms. In this manner, the three axes of the body are moved in a preset exercise pattern. During the series of exercise pattern, the twist motion to be given to the back rising bottom 311 is carried out through an angle of 15° or less with respect to the horizontal plane, and the vertical movement is carried out through an angle of 5° or less with respect to the horizontal plane

Result of experiment of an optimal speed of the vertical movement of the back rising bottom 311 are shown in Fig. 26. The experiment was carried out for seventeen testees, and a speed at which the testees were relaxed was defined as a reclining optimal speed. For the vertical movement speed, i.e., the reclining optimal speed of the back rising bottom 311, the most optimal time was 2.6 times/second, and it was found that more than half testees were relaxed from 2.6 times/second to 4.1 times/second.

According to the relaxing exercise mode 341 of the embodiment, by vertically moving the back rising bottom 311 while twisting the latter in slow rhythm at a speed at which tension is not cause, the three axes of the body are moved and the vertebra erectors are relaxed, parasympathetic nervous wins superiority.

Therefore, muscle tone is moderated, mind is stabilized, stress is eliminated, and relaxed feeling can be obtained.

In the sleeping exercise mode 342, twist motion to be given to the back rising bottom 311 is carried out twice or less/second, the back rising bottom 311 is vertically moved in a predetermined angle range with respect to the horizontal plane while being twisted, and the twist motion and the vertical motion are carried out in predetermined rhythms. In this manner, the three axes of the body are moved in a preset exercise pattern. During the series of exercise pattern, the twist motion to be given to the back rising bottom 311 is carried out through an angle of 20° or less with respect to the horizontal plane, and the vertical movement is carried out through an angle of 20° or less with respect to the horizontal plane. More preferably, the twist motion and vertical movement to be given to the back rising bottom 311 is repeatedly carried out in monotone rhythm while setting a maximum variation of one time to angle of 10° or less. It is preferable that monotone and small motion is repeated in random or 1/f rhythm.

According to the sleeping exercise mode 342 of the embodiment, by vertically moving the back rising bottom 311 while twisting the latter in slow rhythm at a speed at which tension is not cause, parasympathetic nervous wins superiority.

Therefore, monotone is repeated which makes the bed user feel tedious and sleep. In this manner, by promoting sleep using the sleeping exercise, insomnia can be overcome.

In the waking up exercise mode 343, the back rising bottom 311 is moved vertically through a predetermined angle with respect to the horizontal plane while twisting the back rising bottom 311 in rhythm from low speed to high speed.

As variation from low speed to high speed, the rising angle is gradually increased while repeating the vertical motion of the back rising bottom 311 for example.

As another method, twist motion to be given to the back rising bottom 311 is carried out twice or less/second, and then at a speed of twice or less/second or higher.

As another method, twist motion to be given to the back rising bottom 311 is carried out from a speed of twice or less/second to a speed of twice or less/second or higher gradually.

It is also effective that the speed of the twist motion to be given to the back rising bottom 311 is increased stepwisely or continuously, and an angle of the twist motion with respect to the horizontal plane or the angle range of the vertical motion is gradually increased.

According to the waking up exercise mode 343 of the embodiment, by swinging the back rising bottom 311 while varying the rhythm from low speed to high speed, brain is stimulate. If the rising angle is increased or the angle range is increased while repeating the vertical motion of the back rising bottom 311, bloodstream in brain is suppressed and finally, a posture which is easily to rise (blood rise).

Therefore, by gradually increasing stipulation to the brain and gradually increasing the rising angle, the bed user can wake up clearly within a short time by the waking up exercise, and it is safe for a person having encephalopathy or hypotension.

In the finding exercise mode 344, the speed of the twist motion to be given to the back rising bottom 311 is as slow as twice or less/second, thereby allowing the bed user to find small motion of the body, which unleashes original ability that the bed user originally had.

For example, the twist motion and the vertical motion to be given to the back rising bottom 311 are repeated a plurality of times with different timing, or the back rising bottom 311 is driven a plurality of times independently at left and right sides.

According to the finding exercise mode 344 of the embodiment, the bed is slowing moved and the bed user finds a variation of the posture. With this, the bed user's brain finds fine liaison of muscle group around the vertebra erector, and breathing is adjusted.

Therefore, stress of pleural cavity is lessen, breathing is improved, the bed user finds fine motion, which unleashes original ability that the bed user originally had.

In the loin exercise mode 345, the back rising bottom 311 or the leg rising bottom 312 is operated, pressing motion of backbone and releasing motion of sleeping posture are repeated in sitting posture.

In one method, the back rising bottom 311 is moved upward, the three axes of a body are moved upward more than 20° with respect to the horizontal plane around the lateral axis and then, the back rising bottom 311 is lowered while twisting the same, and the three axes of a body are moved in accordance with a preset exercise pattern.

As the preset exercise pattern, the back rising bottom 311 is repeatedly moved upward and downward for a predetermined time. At that time, both or one of the vertical angle range or twisting angle may be increased gradually.

As another method, the back rising bottom 311 and the leg rising bottom 312 are moved upward through a predetermined angle and then, the back rising bottom 311 and the leg rising bottom 312 are lowered while twisting the back rising bottom 311. At that time, a total of the predetermined angle for moving the back rising bottom 311 upward and the predetermined angle for moving the leg rising bottom 312 with respect to the horizontal plane is preferably set to 20° or more. Further, it is preferable to repeat the moving upward motion and moving downward motion of the back rising bottom 311 and the leg rising bottom 312 for a predetermined time. At that time, both or one of the vertical angle range or twisting angle may be increased gradually.

According to the loin exercise mode 345 of the embodiment, the pressing motion of intervertebral disk in a sitting posture, releasing motion of intervertebral disk in a sleeping posture, and stretching motion of psoas, vertebra erector and the like are repeated, the circulation of blood of loin is promoted, and stress is eliminated.

Therefore, the blood stream of loin is increased, loin fatigue is eliminated, and lumbago can be prevented.

Although explanation is omitted in the embodiment, amotion mode for rotating the abdomen of the body lying on the bed through a predetermined angle around a phantom longitudinal axis passing through the abdomen in the longitudinal direction. Since the directional easiness of rotation as viewed from the three axes of the body is different depending upon individuals, it is preferable that the rotation direction is set individually.

By including the rotation around the phantom longitudinal axis passing through the abdomen of the body lying on the bed, the twist as viewed from the three axes, the various exercise patterns are corrected by width of the movable range and easiness of the movable rotational direction, and the various exercise effects are enhanced.

A speed for moving the back rising bottom 311 upward or downward of is preferably selected from preset speeds, or it is preferable to set the speed in a preset speed range.

A speed for moving the leg rising bottom 312 upward or downward of is preferably selected from preset speeds, or it is preferable to set the speed in a preset speed range.

An angle for moving the back rising bottom 311 upward or downward of is preferably selected from preset angles, or it is preferable to set the angle in a preset angle range.

An angle for moving the leg rising bottom 312 upward or downward of is preferably selected from preset angles, or it is preferable to set the angle in a preset angle range.

Preferably, a rotation angle or rotation direction of the back rising bottom 311 or the leg rising bottom 312 is selected from a preset angle or direction, or is set the angle or the direction in a preset angle range.

It is preferable that a plurality of motion patterns comprising a combination of a twisting motion around a phantom vertical axis from the head to a center of both legs of the body lying on a bed , a turning motion around a phantom longitudinal axis passing through the abdomen of the body lying on the bed, and a vertical motion around a phantom lateral axis laterally passing through the abdomen of the body lying on the bedstate are previously set, the order of these motion patterns are changed, or the speed, angle or direction of the motion patterns can be changed.

According to the embodiment, sense of proportion is weaken with aging, an old person is sensitive to speed, and it is necessary to change the speed between young person and old person, but since the moving speed of the bed can freely be changed in accordance with bed user, comfortableness can suitable for individual be supplied, and effects of various exercise can be exhibited.

If each of the above exercises are repeated, the bed user gets used to the motion patters and adapts to the stipulation amount and its effect is reduced, but according to the present embodiment, the effect can be maintained or enhanced by varying the pattern, speed, rotation angle, rotation direction and the like for constant period.

If the rising direction is always the same, there is a possibility that twist is increased, but if the rising direction can be changed as in this embodiment, the twist can be prevented from being increased.

Next, the operating apparatus 320 shown in Fig.25 will be explained.

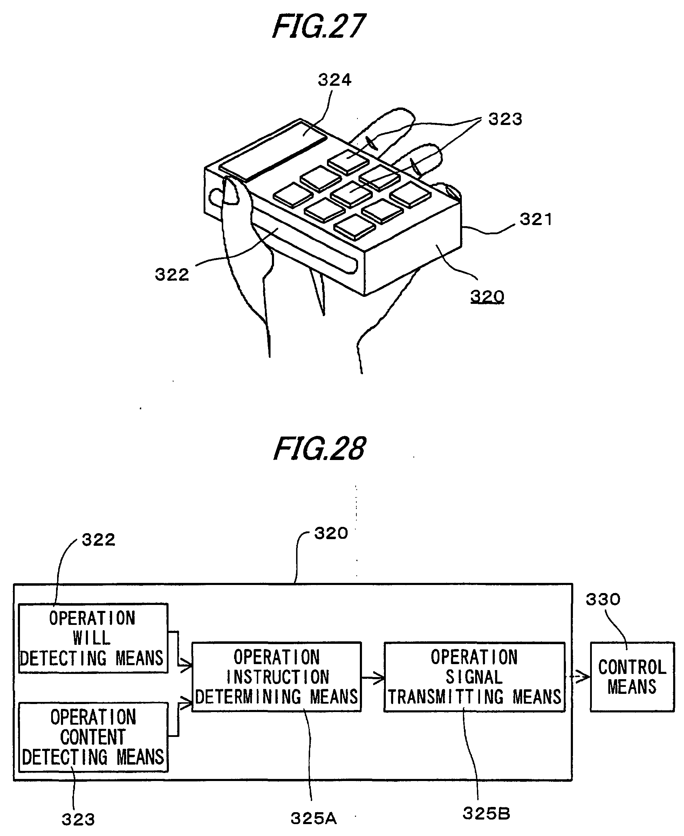

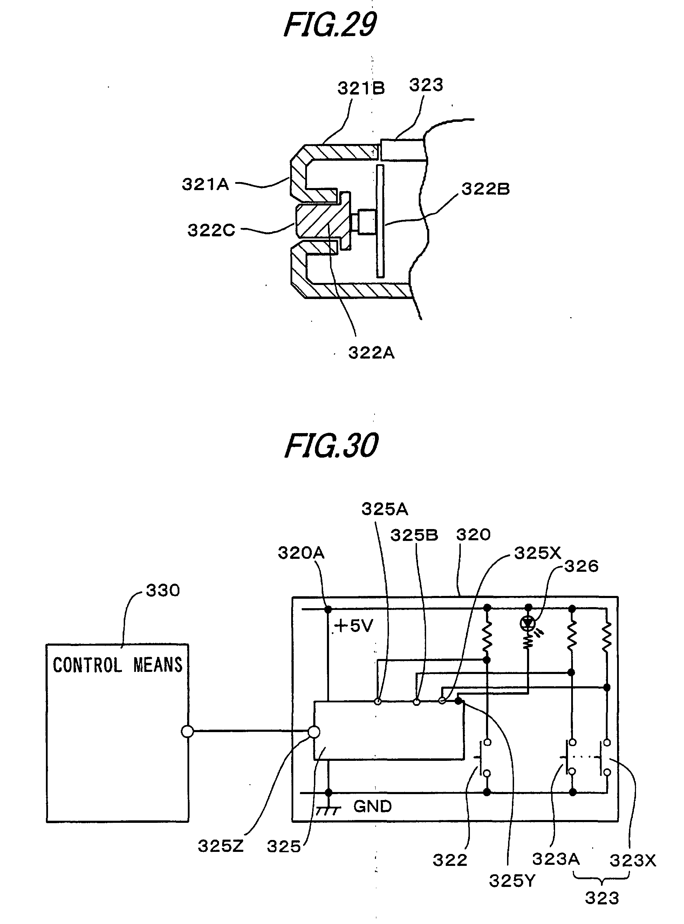

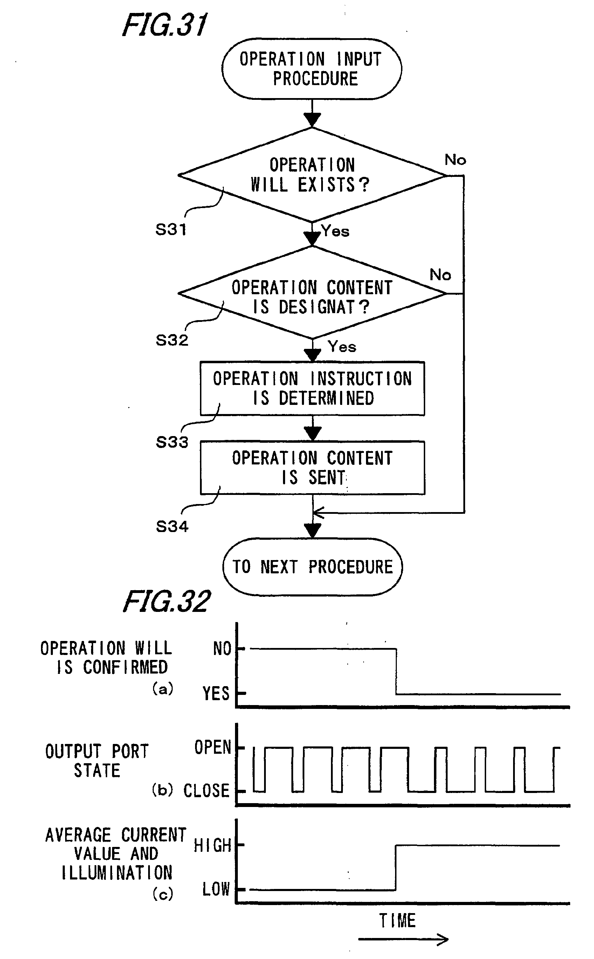

Fig.27 is a perspective view showing the outline structure of the operating apparatus, Fig.28 is a block diagram showing function of the operating apparatus, Fig.29 is a sectional view of an essential portion of the operating apparatus Fig.30 is a circuit diagram showing the outline structure of the operating apparatus, and Fig.31 is a flowchart showing determining process of operation instructions of the operating apparatus.

First, the outline structure of the operating apparatus 320 will be explained using Fig.27.

The operating apparatus 320 has a box-like outline 321 in which operation will detecting means 322 for detecting operating will, operation content detecting means 323 for detecting operation content, and display means 324 for displaying operation content or motion state are provided. Here, the operation will detecting means 322 is provided on a side surface of the outline 321, and the operation content detecting means 323 is provided on a front surface of the outline 321. The display means 324 is provided on the front surface of the outline 321 like the operation content detecting means 323. The operation will detecting means 322 may be provided on each of two opposed side surfaces of the outline 321. When the two operation will detecting means 322 are provided, if one of them is operated to detect the will, the operability is enhanced, and if both of the two must be operated to detect the will, this can prevent erroneous operation.

As shown in Fig.28, processing means 325 comprising operation instruction determiningmeans 325A and operation signal transmitting means 325B is provided in the operating apparatus 320. The operation instruction determining means 325A outputs operation content when the operating will is detected by the operation will detecting means 322 and the operation content is detected by the operation content detecting means 323. The operation signal transmitting means 325B includes a function for transmitting a signal from the operation instruction determining means 325A to the control means 330.

As shown in Fig.29, the operation will detecting means 322 includes a grip detection knob 322A and a grip detection switch 322B. An abutment surface 322C of the grip detection knob 322A is located slightly inner side from the side surface 321A of the outline 321.

As shown in Fig.30, the processing means 325 comprising a one chip microcomputer for example, includes input ports 325A, 325B, 325X, an output port 325Y and a communication port 325Z. The processing means 325, the operation will detecting means 322, the operation content detecting means 323, and LED 326 are connected to a control voltage 320A. Here, in the operation will detecting means 322, the operation content detecting means 323A and 323X, signals are input to the input ports 325A, 325B and 325X in their ON states, but signals from the operation content detecting means 323A, 323X are judged by the processing means 325 as being effective only when a signal from the operation will detecting means 322 is input. The LED 326 is used for illuminate the display means 324 and the operation content detecting means 323, and the illumination can be changed depending upon opening and closing time of the output port 325Y.

Next, the determining process of operating command of the operating apparatus shown in Fig.31 will be explained.

In the input procedure of the operation content, the operation will is judged by the operation will detecting means 322 (S31). If it is judged that there is operation will, it is judged whether the operation by the operation content detecting means 323 is carried out (S32). Here, if the operation content can be confirmed, the operation instructions are determined by the operation instruction determining means 325A (S33), the operation content is sent form the operation signal transmitting means 325B (S34).

As described above, in the operating apparatus 320 of the embodiment, the grip detecting knob 322A is disposed on the side surface 321A of the outline 321, and the abutment surface 322C is disposed at inner side from the side surface 321A and thus, even if the operating apparatus 320 is placed on the bed and the operation content detecting means 323 is pushed by a comforter or the like, the operation will detecting means 322 does not function and thus, the operating apparatus 320 is not operated erroneously against the user's will. Since the operating will is confirmed only when the user grasps the operating apparatus 320, the user can input the operation content without confirming the operating will.

Therefore, according to the embodiment, the operability is enhanced and safe and reliable operation can be carried out without erroneous operation.

Fig.32 is a graph showing the motion state of the operation will ganged illumination.

When the operating will of the LED 326 shown in Fig.30 for the illumination is not confirmed, the closing state of the output port 325Z is changed shorter periodically with respect to the opening state so that the current value flowing through the resistance is reduced relatively. Therefore, when the operating will has not been confirmed, the LED 326 is illuminated with lower illumination. On the other hand, when the operating will has been confirmed, the closing state of the output port 325Z is changed longer periodically with respect to the opening state so that the current value flowing through the resistance is increased relatively. Therefore, when the operating will has been confirmed, the LED 326 is illuminated with higher illumination. Such output port 325Z can be realized by using the one chip microcomputer having a PWM (pulse width modulating) port. After a predetermined time has been elapsed after the operating will was detected by the operation will detecting means 322, and after a predetermined time has been elapsed after the operating will was not detected by the operation will detecting means 322, the illumination may be changed to lower illumination.

If the illumination of the LED 326 is changed based on the operating will, since the illumination of the operating switch is lit even if the room illumination is lowered, sleeping is not prevented.

Thus, according to the embodiment, the operating apparatus is illuminated with high illumination only when the user grasps the operation apparatus, the operability can be enhanced without hindering sleep.

Next, glare moderating illumination means will be explained.

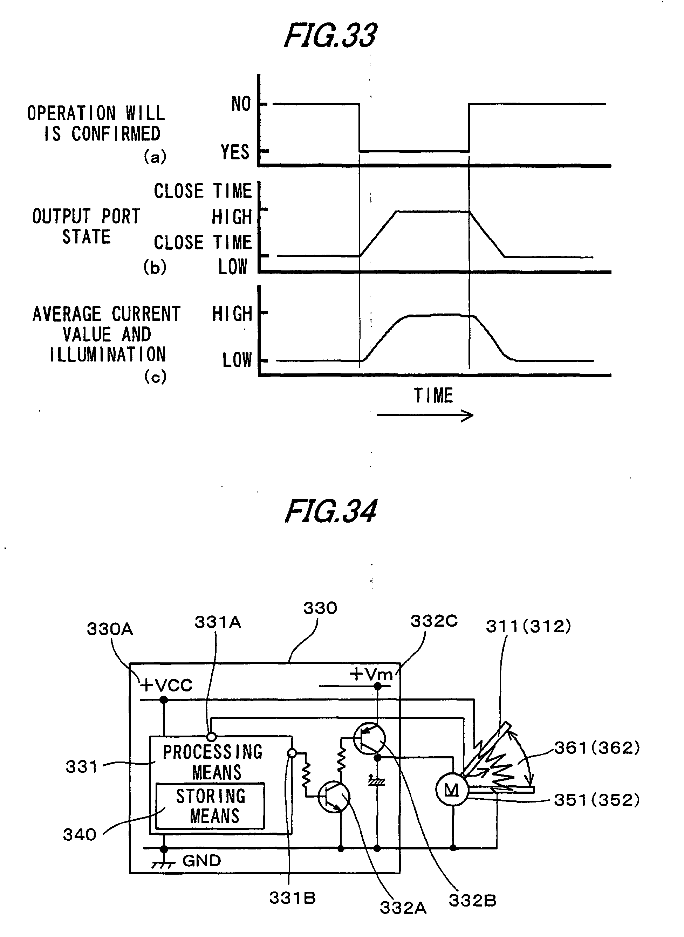

Fig. 33 is a graph showing amotion state of a glare moderating illumination.

When the operating will was confirmed from the non-confirmed state with respect to the illumination LED 326 shown in Fig.30, the opening and closing ratio of the output port 325Z is changed such that the closing time is gradually increased, so that the current value flowing through the resistance is gradually increased. Therefore, when the operating will was confirmed, the illumination for the LED 326 is gradually increased to high level. On the other hand, the operating will was not confirmed from the confirmed state, the opening and closing ratio of the output port 325Z is changed such that the closing time is gradually reduced, so that the current value flowing through the resistance is gradually reduced. Therefore, when the operating will was not confirmed, the illumination for the LED 326 is gradually reduced to low level.

According to the embodiment, since the illumination is gradually changed when the illumination is changed, even if the operation apparatus is used in a state in which the room illumination is lowered when the user sleeps, the illumination is gradually increased, the user is less prone to feel glare, and the user can use the apparatus more comfortably.

The control means 330 shown in Fig.25 will be explained next.

Fig.34 is a circuit diagram showing the outline structure including the control means.

As shown in Fig.34, the control means 330 comprises processing means 331 comprising a one chip microcomputer for example, and voltage amplifying transistors 332A and 332B. The processing means 331 includes storing means 340, an A/D input port 331A and a D/A output port 331B. The processing means 331 is connected to a control voltage 330A, and the voltage amplifying transistors 332A and 332B are connected to a control voltage 332C. The position detecting means 361 (362) is connected to the A/D input port 331A of the processing means 331, and the back rising driving means 351 (or leg rising driving means 352) is connected to the D/A output port 331B.

The storing means 340 previously stores information of target positions of a series of motions, sequentially reads out the stored target position when the motion is reproduced, and the back rising driving means 351 (or the leg rising driving means 352) is operated as a motion target position of the next time.

When the driving direction is reversed, a voltage reversing circuit using a relay part or the like for switching the control voltage to the opposite pole (changing + and -) of control voltage to be applied to the back rising driving means 351 (or the leg rising driving means 352) is provided.

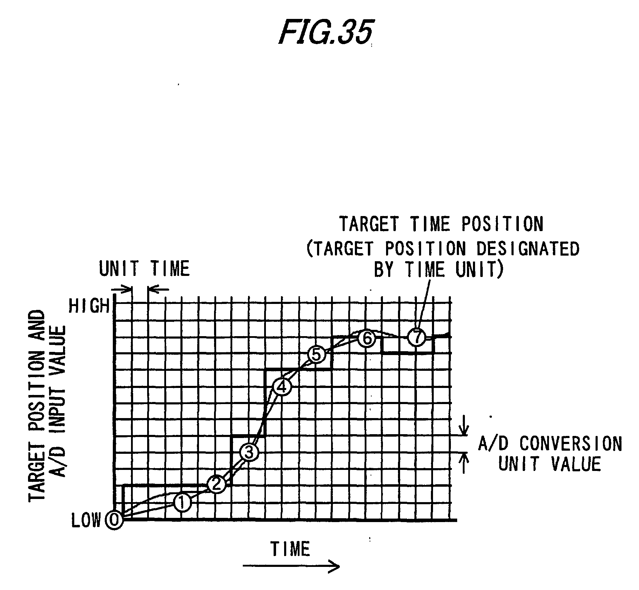

Fig.35 is a graph showing motion of a continuous driving control means.

Fig.35 shows a value of an actual position detected by the position detecting means 361 (362) and an A/D value after A/D conversion at apparently the same values. The control is conducted such that the order of the actual time unit is millisecond order, and resolving power of the actual position confirmation is 1 mm (or 1°) or less, and control resolving power of A/D and D/A conversion unit value is 256 to 512 steps, and the motion feeling that the actual user feels is linear driving.

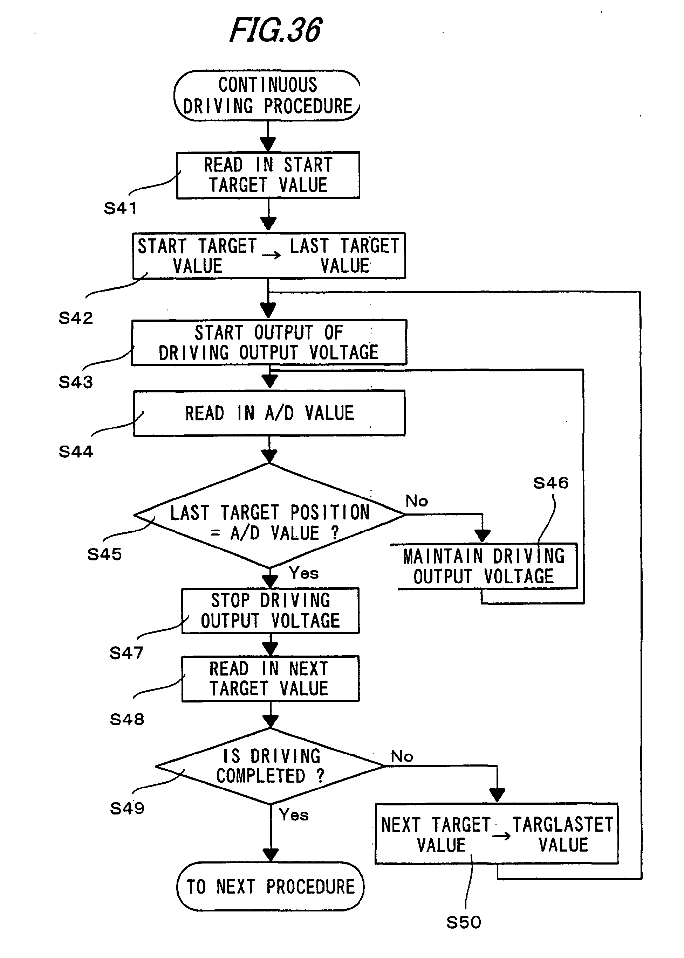

Fig.36 is a flowchart showing the continuous driving processing.

The continuous driving processing will be explained using Fig.36.

In the continuous driving processing, a start target value (2 in Fig.35) is read in from the storing means 340. Next, the start target value (2 in Fig.35) and a last target value (1 in Fig. 35) are compared with each other, and based on the comparison result, output of the driving voltage from the D/A output port 331 B is started (S43). Next, A/D value input from the position detecting means 361 (362) to the A/D input port 331A is read in (S44). The read A/D value and an A/D value at the last target value (1 in Fig.35) are compared with each other (S 45), and if they are equal to each other, the driving output is stopped (S47), and a next target value (3 in Fig.35) is read in (S48). If they are not equal to each other, the driving output voltage is maintained (S46), and the A/D value is again read in (S44). The driving completion is judged depending upon whether the next target exists (S49), and if there is the next target value, the next target value (3, in Fig.35) and the last target value (2 in Fig.35) are compared with each other (S50), and the output of the driving voltage from-the D/A output port 331B is started based on the comparison result.

According to the embodiment, the target portion to be moved can be driven toward the target position sequentially at each motion time point with respect to the series of previously set target positions (angle) and thus, each moving position detected by the position detecting means can be brought into synchronization with previously stored target values. Thus, various continuous motions on the bed motions can be constituted as program, and various motions can be set.

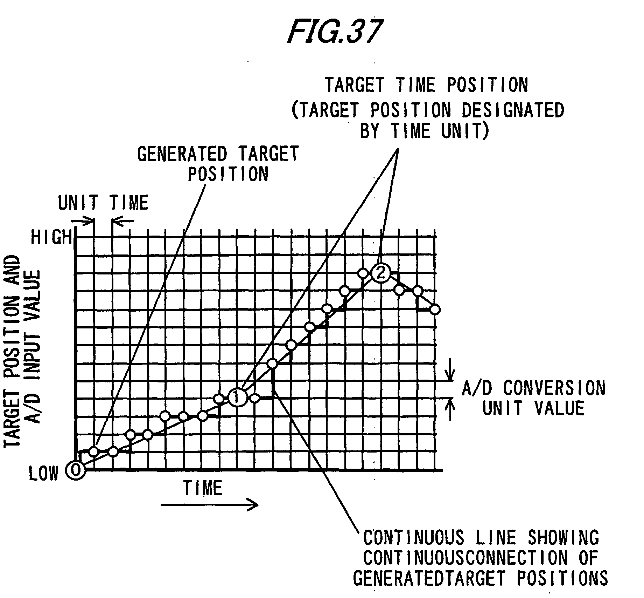

Fig.37 is a graph showing a principle of a section speed control method.

Fig.37 shows a value of an actual position detected by the position detecting means 361 (362) and an A/D value after A/D conversion at apparently the same values.

The continuous target positions are calculated (moving position (distance or angle) per unit time is calculated from inclination) per unit time from position information in a section (e.g., a section between 1 and 2) of two continuous target positions, and a deviation of the actual motion position read from the A/D input value with respect to a generated target position of each elapsed time unit is calculated as a generated target position. If the actual motion position did not reach the generated target position, the D/A output value for controlling the power supply voltage for driving the back rising driving means 351 (or leg rising driving means 352) is added stepwisely, thereby increasing the voltage, and the delay is compensated. If the generated target position exceeds the actual motion position, the D/A output value is stepwisely subtracted to lower the voltage, so that the excessive value is cut off. In this manner, the section motion speed is made constant, and target portions can be moved in the order of the target time positions.

According to the embodiment, the target values are sequentially read out, continuous generated target values are calculated per predetermined unit time by the position information between two continuous target positions, each the detected moving position and each the generated target value are compared to each other to drive the back rising driving means 351 (or leg rising driving means 352) so that the motion can be brought into synchronization with the generated target position obtained by dividing the motion target section further finely by the constant time interval and thus, optimal moving speed set when it is designed can always be kept.

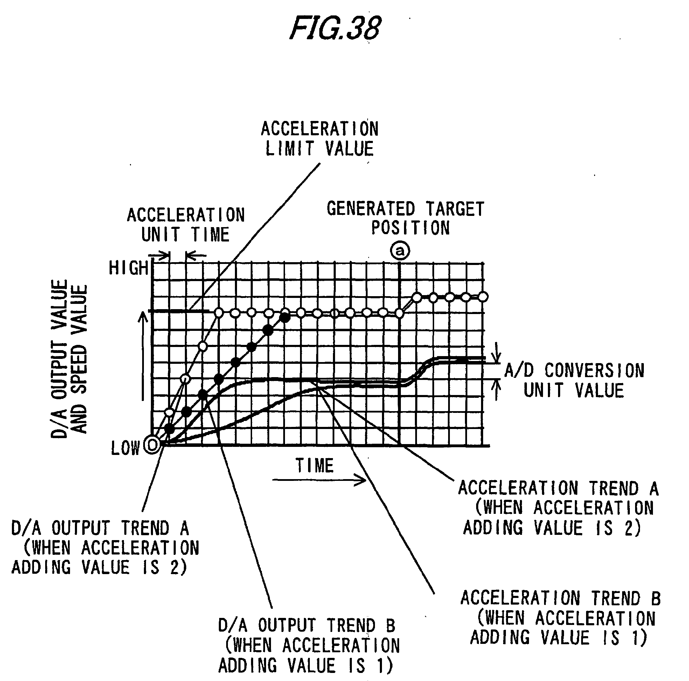

Fig.38 is a graph showing a motion state at the time of acceleration of an impact absorbing driving means.

As shown in Fig.38, the unit time is divided into further fine unit times at the time of acceleration and deceleration as acceleration and deceleration unit time, the acceleration adding value is added per distance of the acceleration and deceleration unit time up to an acceleration limit value which is a pre-defined D/A output value, and the speed is added sequentially. Thus, as compared with a case in which the acceleration adding value is 2, when the acceleration adding value is 1, variation per time of the voltage apply to the back rising driving means 351 (or leg rising driving means 352) becomes gentle, the trend of speed state is gentle and thus, the user is less prone to feel impact.

Further, after the acceleration, the control is shifted from the deviation between the target position and the actual motion position at the time of the first generated target position (of unit time designation: time point a in the drawing) to control of the D/A output value by the action of the section speed control means, and the target portion can be moved in a state in which the section speed is controlled. (The drawing shows a state in which the current position did not reach the target position and thus, the D/A output value was added). Therefore, it is possible to control arbitrary acceleration speed by designating the acceleration adding value when the motion is started, and acceleration can be controlled based on designating motion without impact.

According to the embodiment as described above, when the back rising driving means 351 (or leg rising driving means 352) is started or stopped, voltage to be applied to the back rising driving means 351 (or leg rising driving means 352) is stepwisely changed based on preset arbitrary acceleration variation value and thus, acceleration can be controlled without impact.

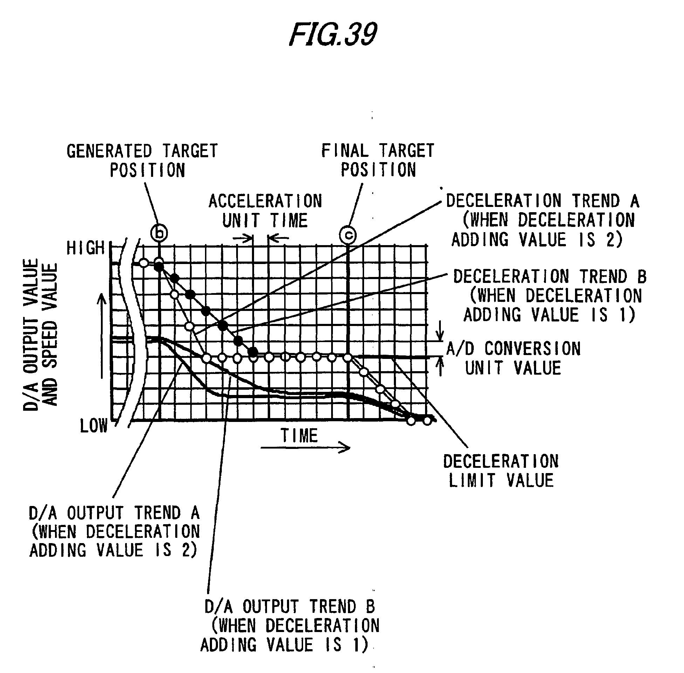

Fig.39 is a graph showing a motion state at the time of deceleration of the impact absorbing driving means.

As shown in Fig.39, at the time of deceleration, after the target portion passed through the last generated target position (b, in the drawing), the deceleration subtracting value is subtracted in each distance of the acceleration deceleration unit time up to an deceleration limit value which is a pre-defined D/A output value to sequentially decelerate. Thus, as compared with a case in which the deceleration subtracting value is 2, when it is 1, variation per time of the voltage apply to the driving means becomes gentle, the trend of speed state is gentle and thus, the user-is less prone to feel impact. Next, after the deceleration limit value is reached, until the last target position (c, in the drawing) is reached, the D/A output value of the deceleration limit value is kept, and the driving means is operated. With this, the position of the final target value can be detected correctly, and when the final target position is reached, the D/A output value in each distance of the acceleration deceleration unit time is subtracted one unit base, and the apparatus is substantially stopped completely when the D/A output value becomes zero. In order to bring the stop time close to the final target position more correctly, it is necessary that the moving speed of the target portion in the D/A output value of the deceleration limit value is sufficiently slow. Therefore, a value of the deceleration limit value is determined while confirming using an actual apparatus while taking precision of the stop position and sensed delay required to stop into consideration.

According to the embodiment, the back rising driving means 351 (or leg rising driving means 352) can move based on preset arbitrary acceleration variation value. Therefore, acceleration which is set into a constant level can be realized, irrespective of using condition.

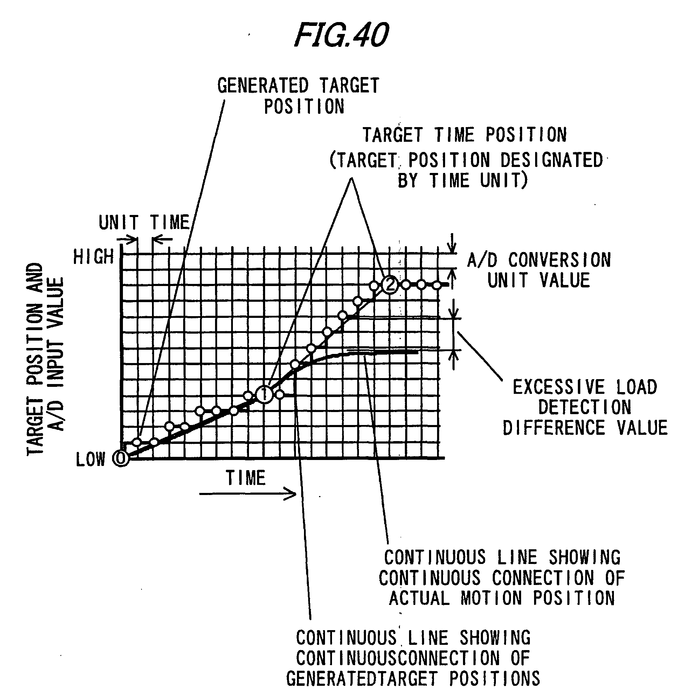

Fig.40 is a graph showing a principle of the excessive load detecting means.

Fig.40 shows a value of an actual position and an A/D value after A/D conversion at apparently the same values.

As shown in Fig.40, when the motion position in an actual moving direction is deviated from a generated target position on control by an excessive load detection difference value, this state is judged as an excessive load state, the D/A output value is stopped, and the back rising driving means 351 (or leg rising driving means 352) is stopped. With this, it becomes possible to detect that excessive load equal to or greater than driving ability of the back rising driving means 351 (or leg rising driving means 352) was applied, it is possible to provide a safe bed inexpensively without adding parts (overcurrent detecting circuit or distortion detecting part).

According to the embodiment, when a difference between a target value and a detected moving position exceeds the preset excessive load detection difference value, this state is judged as the excessive load state. Therefore, the bed can be prevented from being destroyed in the excessive load state without requiring separate adding parts.

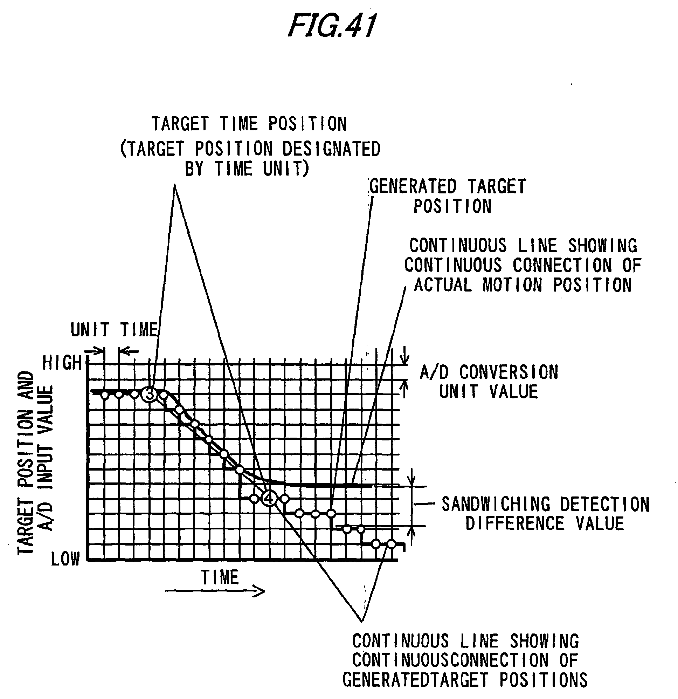

Fig.41 is a graph showing a principle of the sandwich detecting means.

Fig. 41 shows a value of an actual position and an A/D value after A/D conversion at apparently the same values.

As shown in Fig.41, when a deviation between a motion position in a direction opposite from the actual motion and a generated target position on control equal to or greater than a sandwiching detection difference value is generated, this state is judged as a sandwiching state, the D/A output value is stopped, and the back rising driving means 351 (or leg rising driving means 352) is stopped. With the above operation, it is possible to detect that the sandwiching is generated on control and thus, it is possible to provide a safe bed inexpensively without adding parts. When the sandwiching is detected, if the target portion is driven in a sandwiching-releasing direction for a constant time in addition to the stop of the driving means, further safety is provided of course.

As described above, according to the embodiment, since the sandwiching is judged from the moving position of the driving means and the deviation of in the opposite direction of an actual position, it is unnecessary to add the separate parts, and it is possible to provide a sandwiching preventing function when there is a possibility that sandwiching constituted by driving target portions.

### INDUSTRIAL APPLICABILITY

As apparent from the above embodiments, according to the present invention provides a rational apparatus and a method for moving a human body, and an adjusting method and an adjusting apparatus for spending comfortable and healthy life which are safe and comfortable to anyone including old persons, and which does not apply excessive force to the body in which, based on natural motions around three phantom axes of human body, one of the motion or a combination of the motions are regularly carried out.

## Claims

1. An apparatus for moving a body, wherein a phantom axis passing a head to a center of legs of the body is defined as a vertical axis, a phantom axis passing an abdomen in the longitudinal direction is defined as a longitudinal axis, and a phantom axis passing the abdomen in the lateral direction is defined as a lateral axis, and motions around these three axes are carried out individually or in combination.

2. An apparatus for moving a body, wherein a phantom axis passing a head to a center of legs of the body is defined as a vertical axis, and a phantom axis passing an abdomen in the longitudinal direction is defined as a longitudinal axis, and motions around these two axes are carried out individually or in combination.

3. An apparatus for moving a body, wherein a phantom axis passing a head to a center of legs of the body is defined as a vertical axis, and a phantom axis passing the abdomen in the lateral direction is defined as a lateral axis, and motions around these two axes are carried out individually or in combination.

4. An apparatus for moving a body, wherein a phantom axis passing an abdomen in the longitudinal direction is defined as a longitudinal axis, and a phantom axis passing the abdomen in the lateral direction is defined as a lateral axis, and motions around these two axes are carried out individually or in combination.

5. An apparatus for moving a body, wherein a phantom axis passing a top of a head to soles of legs of the body standing upright or lying face up or down is defined as a vertical axis, a phantom axis passing an abdomen in the longitudinal direction is defined as a longitudinal axis, and a phantom axis passing the abdomen in the lateral direction is defined as a lateral axis, and a motions around these three phantom axes are regularly carried out.

6. An apparatus for moving a body, wherein a phantom axis passing a top center of a head to a center of soles of legs of the body standing upright or lying face up or down is defined as a vertical axis, a phantom axis passing a bendable abdomen in the longitudinal direction is defined as a longitudinal axis, and a phantom axis passing the bendable abdomen in the lateral direction is defined as a lateral axis, and a turning motion around said vertical axis, a laterally swinging motion around said longitudinal axis and a lying up and down motion around said lateral axis are combined.

7. An apparatus for moving a body, wherein a phantom axis passing a top center of a head to a center of sitting posture of the sitting body is defined as a vertical axis, a phantom axis passing an abdomen in the longitudinal direction is defined as a longitudinal axis, and a phantom axis passing the abdomen in the lateral direction is defined as a lateral axis, and motions around these three phantom axes are regularly carried out.

8. An apparatus for moving a body, wherein a phantom axis passing a top center of a head to a center of sitting posture of the sitting body is defined as a vertical axis, a phantom axis passing a bendable abdomen in the longitudinal direction is defined as a longitudinal axis, and a phantom axis passing the bendable abdomen in the lateral direction is defined as a lateral axis, and a turning motion around said vertical axis, a laterally swinging motion around said longitudinal axis, and a lying up and down motion around said longitudinal axis are combined.

9. An apparatus for moving a body, wherein a phantom axis passing through a head to a center of legs of the body is defined as a vertical axis, a phantom axis passing through an abdomen surface to a back surface in a longitudinal direction is defined as a longitudinal axis, and a phantom axis passing through a body side surface in a lateral direction is defined as a lateral axis, a rotating motion around said vertical axis based on at least one origin from a lumbar vertebrae, a chest thoracic vertebrae and a cervical vertebra, a laterally swinging motion around a phantom axis in which a position of said longitudinal axis is at least one point from a loin to a chest and cervix, and a lying up and down motion around a phantom axis in which a position of said lateral axis is at'least one point from the loin to the chest and the cervix, are carried out individually or in combination.

10. An apparatus for moving a body according to claim 9, wherein when at least two of said turning motion, said laterally swinging motion and said lying up and down motion are carried out continuously, a position of an origin of said vertical axis, a position of said longitudinal axis and a position of said lateral axis are fixed or moved.

11. A bed for moving a body using an apparatus for moving a body described in any one of claims 1 to 10.

12. A body adjusting method, wherein a phantom axis passing a head to a center of legs of the body is defined as a vertical axis, a phantom axis passing an abdomen in the longitudinal direction is defined as a longitudinal axis, and a phantom axis passing the abdomen in the lateral direction is defined as a lateral axis, and motions around these three axes are carried out individually or in combination, and the method adjusts the body so that distortion or unbalance of the body is eliminated, circulation of blood is promoted, and the body is relaxed.

13. A body adjusting method, wherein a phantom axis passing a top of a head to soles of legs of the body standing upright or lying face up or down is defined as a vertical axis, a phantom axis passing an abdomen in the longitudinal direction is defined as a longitudinal axis, and a phantom axis passing the abdomen in the lateral direction is defined as a lateral axis, and a motions around these three phantom axes are regularly carried out so that distortion or unbalance of the body is eliminated, circulation of blood is promoted, and the body is relaxed.

14. A body adjusting method, wherein a phantom axis passing a top center of a head to a center of soles of legs of the body standing upright or lying face up or down is defined as a vertical axis, a phantom axis passing a bendable abdomen in the longitudinal direction is defined as a longitudinal axis, and a phantom axis passing the bendable abdomen in the lateral direction is defined as a lateral axis, and a turning motion around said vertical axis, a laterally swinging motion around said longitudinal axis and a lying up and down motion around said lateral axis are combined so that distortion or unbalance of the body is eliminated, circulation of blood is promoted, and the body is relaxed.

15. A body adjusting method, wherein a phantom axis passing a top center of a head to a center of sitting posture of the sitting body is defined as a vertical axis, a phantom axis passing an abdomen in the longitudinal direction is defined as a longitudinal axis, and a phantom axis passing the abdomen in the lateral direction is defined as a lateral axis, and motions around these three phantom axes are regularly carried out so that distortion or unbalance of the body is eliminated, circulation of blood is promoted, and the body is relaxed.

16. A body adjusting method, wherein a phantom axis passing a top center of a head to a center of sitting posture of the sitting body is defined as a vertical axis, a phantom axis passing a bendable abdomen in the longitudinal direction is defined as a longitudinal axis, and a phantom axis passing the bendable abdomen in the lateral direction is defined as a lateral axis, and a turning motion around said vertical axis, a laterally swinging motion around said longitudinal axis, and a lying up and down motion around said lateral axis are combined so that distortion or unbalance of the body is eliminated, circulation of blood is promoted, and the body is relaxed.

17. A body adjusting method, wherein a phantom axis passing through a head to a center of legs of the body is defined as a vertical axis, a phantom axis passing through an abdomen surface to a back surface in a longitudinal direction is defined as a longitudinal axis, and a phantom axis passing through a body side surface in a lateral direction is defined as a lateral axis, a rotating motion around said vertical axis based on at least one origin from a lumbar vertebrae, a chest thoracic vertebrae and a cervical vertebra, a laterally swinging motion around a phantom axis in which a position of said longitudinal axis is at least one point from a loin to a chest and cervix, and a lying up and down motion around a phantom axis in which a position of said longitudinal axis is at least one point from the loin to the chest and the cervix, are carried out individually or in combination so that distortion or unbalance of the body is eliminated, circulation of blood is promoted, and the body is relaxed.

18. A body adjusting method according to claim 17, wherein a turning motion around said vertical axis having an origin which is at least one of the loin, the chest and cervix, a laterally swinging motion around a phantom axis having a position of said longitudinal axis which is at least one of the loin, the chest and cervix, and a lying up and down motion around a phantom axis having a position of said longitudinal axis which is at least one of the loin, the chest and cervix, are carried out individually or in combination, and when two or more of said body motions are carried out continuously, a position of said origin of said vertical axis, a position of said longitudinal axis and a position of said lateral axis are fixed or moved every time, so that distortion or unbalance of the body is eliminated, circulation of blood is promoted, and the body is relaxed.

19. An adjusting apparatus for carrying out a body adjusting method described in any one of claims 12 to 18.

20. A bed having a bottom which is divided into a plurality of members and in which the divided members can move such as to form different slants, wherein said bed includes swinging mechanism means for giving a twisting motion to at least one of said members of said bottom.

21. A bed according to claim 20, wherein the member of said bottom to which the twisting motion is given by said swinging mechanism means is a back rising bottom for supporting a back.

22. A bed according to claim 20 or 21, wherein an inclination angle of a back rising bottom for supporting a back can be changed.

23. A bed according to claim 20, wherein the member of said bottom to which the twisting motion is given by said swinging mechanism means is a leg rising bottom for supporting a leg.

24. A bed according to claim 20 or 23, wherein an inclination angle of a leg rising bottom for supporting a leg can be changed.

25. A bed having a bottom which is divided into a plurality of members and in which the divided members can move such as to form different slants, wherein at least one of the members of said bottom constitutes a back rising bottom, at least one of the members of said bottom constitutes a leg rising bottom, and inclination angles of said back rising bottom and leg rising bottom can be changed.

26. A bed according to claim 25, wherein at least two of the members of said bottom constitute the leg rising bottom, and at least two of the members constituting the leg rising bottom form a mountain-like slant such as to bring a knee of a body.

27. A bed according to claim 25, further comprising back rising driving means for changing the inclination angle of said back rising bottom, and leg rising driving means for changing the inclination angle of said leg rising bottom.

28. A bed according to claim 27, wherein said back rising driving means changes an inclination angle of said back rising bottom while twisting the back rising bottom.

29. A bed according to claim 27, wherein said leg rising driving means changes an inclination angle of said leg rising bottom while twisting the leg rising bottom.

30. A bed according to claim 27, further comprising display means for displaying a twist angle or inclination angle of said back rising bottom or said leg rising bottom.

31. A bed according to claim 27, further comprising an operating portion for controlling a driving operation of said back rising driving means and said leg rising driving means.

32. A bed according to claim 27, further comprising storing means for storing a twist angle or an inclination angle of said back rising bottom or said leg rising bottom.

33. A bed comprising a bottom which is divided into a plurality of members which can move such as to form different slants, a body motion detector for detecting a motion of a body existing on said bottom, sleeping state judging means for judging a sleeping state by a signal from said body motion detector, driving means for moving at least one member of said bottom, and driving control means for controlling a driving of said driving means, wherein said driving control means controls the driving of said driving means by a signal from said sleeping state judging means.

34. A bed according to claim 33, wherein said driving control means moves said driving means after a predetermined time is elapsed from an instant when said driving control means received a sleeping state completion judging signal from said sleeping state judging means.

35. Abed according to claim 33, wherein if said driving control means received a sleeping state judging signal from said sleeping state judging means, said driving control means allows said driving means to move such as to return the back rising bottom and the leg rising bottom to a horizontal state or an inclination state which was previously set as a slant in the sleeping state

36. A bed comprising a bottom which is divided into a plurality of members which can move such as to form different slants, a twisting driving means for twisting at least one of the members of said bottom, a plurality of load sensors for detecting weights of portions of a body existing on said bottom, a plurality of output means for outputting a detection signal detected by said load sensor, and comparing means for comparing output signal value from said output means, wherein said twisting driving means twists at least one of the members of said bottom based on a comparison signal from said comparing means.

37. A bed comprising a bottom which is divided into a plurality of members which can move such as to form different slants, a driving means for moving at least one of the members of said bottom, a load sensor for detecting a weight of a body existing on said bottom, output means for outputting a detection signal detected by said load sensor, and a time setting means for setting a predetermined time, wherein when a signal is not output from said output means within time set by said time setting means, this state is judged as a sleeping state, and an output signal from said driving means is changed.

38. , A bed according to claim 37, wherein when a signal is not output from said output means within time set by said time setting means, a motion state is released, or the state is returned to a state which was previously set as a sleeping state.

39. A bed comprising a bottom which is divided into a plurality of members which can move such as to form different slants, a twisting driving means for moving at least one of the members of said bottom, and timer means for setting a predetermined time, wherein after time set by the timer means was elapsed, an output signal from said driving means is changed.

40. , A bed according to claim 39, wherein after time set by the timer means was elapsed, a motion state is released, or the state is returned to a state which was previously set as a sleeping state.

41. A bed comprising a bottom which is divided into a plurality of members which can move such as to form different slants, a driving means for moving at least one of the members of said bottom, a load sensor for detecting a weight of a body existing on said bottom, output means for outputting a detection signal detected by said load sensor, and weight judging means for judging whether there is a body based on a signal from said output means, wherein when the existence of said body can not be confirmed by said weight judging means, said driving means is not moved.

42. A bed according to claim 41, further comprising weight setting means for storing weight of a bed user, wherein said weight judging means judges the bed user from a weight detected by said load sensor and a weight set by said weight setting means, and when said weight judging means can not confirm the bed user, said driving means is not moved.

43. A bed comprising a bottom divided into a plurality of members, twisting mechanism means for twisting a back rising bottom supporting a back of said bottom, a plurality of load sensor disposed between legs of said bottom and a floor surface, weight judging means for calculating a weight of each portion based on a detection signal detected by said load sensors, weight comparing means for comparing weights of portions calculated by said weight judging means, deviation amount converting means for converting a weight difference obtained by comparison of said weight comparing means into a deviation amount with respect to a center of said bed, twisting motion correcting means for correcting a twisting amount based on said deviation amount, and driving means for driving said twisting mechanism means based on twist motion corrected by said twisting motion correcting means.

44. A bed driving method having a back rising bottom for supporting a back, wherein said back rising bottom is vertically moved in a predetermined angle range with respect to a horizontal plane while twisting said back rising bottom.

45. A bed driving method according to claim 44, wherein a twist motion and a vertical motion given to said back rising bottom are carried out in predetermined rhythm.

46. A bed driving method according to claim 44, wherein a twist motion given to said back rising bottom is carried out twice or less/second.

47. A bed driving method according to claim 44, wherein a twist motion given to said back rising bottom is carried out at an angle of 15° or more at maximum with respect to a horizontal plane, and a vertical motion is carried out at an angle of 5° at maximum with respect to the horizontal plane.

48. A bed driving method according to claim 44, wherein a twist motion and a vertical motion given to said back rising bottom are repeatedly carried out while setting a maximum variation of one time is equal to or less than 10°.

49. A bed driving method according to claim 44, wherein a twist motion given to said back rising bottom is carried out at an angle of 20° or less with respect to a horizontal plane, and a vertical motion is carried out at an angle of 20° or less with respect to the horizontal plane.

50. A bed driving method according to claim 44, wherein a twist motion given to said back rising bottom is carried out at a speed of twice or less/second for a predetermined time-and then, the twist motion is carried out at a speed higher than twice/second.

51. A bed driving method according to claim 44, wherein a twist motion given to said back rising bottom is carried out while gradually increasing speed from twice or less/second to twice or more/second.

52. A bed driving method according to claim 44, wherein an angle of a twist motion with respect to a horizontal plane and an angle of a vertical motion with respect to a horizontal plane are gradually increased.

53. A bed driving method according to claim 44, wherein a twist motion and a vertical motion given to said back rising bottom are carried out at different timings.

54. A bed driving method having a back rising bottom for supporting a back, wherein said back rising bottom is brought upward through a predetermined angle and then, said back rising bottom is lowered while twisting said back rising bottom.

55. A bed driving method according to claim 54, wherein said predetermined angle for bringing said back rising bottom upward is 20° or more with respect to a horizontal plane.

56. A bed driving method according to claim 54, wherein said back rising bottom is repeatedly brought upward and downward.

57. A bed driving method having a back rising bottom for supporting a back and a leg rising bottom for supporting a leg, wherein said back rising bottom and said leg rising bottom are brought upward through a predetermined angle and then, said back rising bottom and said leg rising bottom are lowered while twisting said back rising bottom.

58. A bed driving method according to claim 57, wherein a total of said predetermined angle for bringing said back rising bottom and said predetermined angle for bringing said leg rising bottom with respect to a horizontal plane is 20° or more.

59. A bed driving method according to claim 57, wherein said back rising bottom and said leg rising bottom are repeatedly brought upward and downward.

60. A bed driving method according to any one of claims 44 to 59, wherein a speed for bringing said back rising bottom upward or downward can be selected from preset speeds, or can be set in a preset speed range.

61. A bed driving method according to any one of claims 57 to 59, wherein a speed for bringing said leg rising bottom upward or downward can be selected from preset speeds, or can be set in a preset speed range.

62. A bed driving method according to any one of claims 44 to 59, wherein an angle for bringing said back rising bottom upward or downward can be selected from preset angles, or can be set in a preset angle range.

63. A bed driving method according to any one of claims 57 to 59, wherein an angle for bringing said leg rising bottom upward or downward can be selected from preset angles, or can be set in a preset angle range.

64. A bed driving method having a back rising bottom for supporting a back or a leg rising bottom for supporting a leg, wherein said back rising bottom or said leg rising bottom is rotated through a predetermined angle around a phantom longitudinal axis passing an abdomen of a body lying on a bed in a longitudinal direction.

65. A bed driving method according to claim 64, wherein a rotation angle or a rotation direction of said back rising bottom or said leg rising bottom can be selected from preset angles, or can be set in a preset angle range.

66. A bed driving method having a back rising bottom for supporting a back and a leg rising bottom for supporting a leg, wherein said back rising bottom and said leg rising bottom carry out a twist motion around a phantom vertical axis passing through a head and a center of legs of a body lying on a bed, a turning motion around a phantom longitudinal axis passing through an abdomen of the body lying on the bed in a longitudinal direction, and a vertical motion around a phantom lateral axis passing through the abdomen of the body lying on the bed in a lateral direction, a plurality of motion patterns comprising said twist motion, said turning motion and said vertical motion individually or in combination are previously set, the order of these motion patterns is changed, or a speed, an angle or a direction of each of said motion patterns can be changed.

67. A bed operating apparatus comprising a box-like outline, operation will detecting means provided on at least one surface of said outline for detecting operation will, operation content detecting means provided on at least one surface of said outline for detecting operation content, and operation instruction determining means for outputting said operation content when operation will is detected by said operation will detecting means and operation content is detected by said operation content detecting means.

68. A bed operating apparatus according to claim 67, wherein said operation will detecting means is provided on each of two opposed side surfaces of said outline.

69. A bed operating apparatus according to claim 67, wherein said operation will detecting means and said operation content detecting means are provided on different surfaces of said outline.

70. A bed operating apparatus according to claim 67, further comprising display means for displaying the operation content or motion state of the bed, and illumination changing means for changing illumination of said display means, wherein said illumination changing means changes illumination to high level as compared before the operation will is detected, when the operating will is detected by said operation will detecting means.

71. A bed operating apparatus according to claim 70, wherein after a predetermined time was elapsed from an instant when the operating will was detected by said operation will detecting means, or after a predetermined time was elapsed from an instant when the operating will was not detected by said operation will detecting means, or when the operating will was not detected by said operation will detecting means, the illumination is changed to low level.

72. A bed operating apparatus according to claim 70 or 71, wherein said illumination is gradually changed.

73. A bed driving apparatus comprising storing means which previously stores moving positions of target portions and reaching time to each of the moving positions as target values, driving means for moving said target portions, and position detecting means for detecting motion positions of the target portions by said driving means, wherein the motion positions detected by said position detecting means are brought into synchronism with target values previously stored, said target values are sequentially read out, each detected motion position and each target value are compared to drive said driving means.

74. A bed driving apparatus comprising storing means which previously stores moving positions of target portions and reaching time to each of the moving positions as target values, driving means for moving said target portions, and position detecting means for detecting motion positions of the target portions by said driving means, wherein the motion positions detected by said position detecting means are brought into synchronism with target values previously stored, said target values are sequentially read out, generated target values which are continuous are calculated per unit time which is previously determined by position information between two continuous target positions, the detected motion position and each generated target value are compared to drive said driving means.

75. A bed driving apparatus according to claim 73 or 74, wherein when said driving means is started or stopped, voltage to be applied to said driving means is stepwisely changed based on preset arbitrary acceleration variation value.

76. A bed driving apparatus according to claim 73 or 74, wherein when a difference between said target value and a detected moving position exceeds a preset excessive load detection difference value, this state is judged as an excessive load state.

77. A bed driving apparatus according to claim 73 or 74, wherein when a difference between said target value and a detected moving position exceeds a preset sandwiching detection difference value, this state is judged as a sandwiching state.
